# EUROPEAN PATENT SPECIFICATION

(11) **EP 3 562 946 B1**
(45) Date of publication and mention of the grant of the patent: **24.11.2021**
(21) Application number: 17826228.3
(22) Date of filing: 21.12.2017
(51) Int. Cl.: C12N 15/86, A61K 35/76, C12N 15/87, C12N 9/78

(54) **ONCOLYTIC VIRUSES AND THERAPEUTIC MOLECULES**
ONKOLYTISCHE VIREN UND THERAPEUTISCHE MOLEKÜLE
VIRUS ONCOLYTIQUES ET MOLÉCULES THÉRAPEUTIQUES

(30) Priority: 28.12.2016 EP 16306831
(43) Date of publication of application: 06.11.2019
(73) Proprietor: Transgene SA, 67400 Illkirch Graffenstaden (FR)
(72) Inventor: ERBS, Philippe, 67120 Molsheim (FR); FOLOPPE, Johann, 67115 Plobsheim (FR)
(74) Representative: Vossius & Partner Patentanwälte Rechtsanwälte mbB
(86) International application number: PCT/EP2017/084022
(87) International publication number: WO 2018/122088

(56) References cited:
- WO-A1-2010/118013
- FEND LAETITIA ET AL: "Oncolytic virotherapy with an armed vaccinia virus in an orthotopic model of renal carcinoma is associated with modification of the tumor microenvironment", ONCOIMMUNOLOGY, LANDES BIOSCIENCE, US, vol. 5, no. 2, E1080414, 1 January 2016 (2016-01-01), pages 1-11, XP008180456, ISSN: 2162-402X
- KREMER MELANIE ET AL: "Vaccinia virus replication is not affected by APOBEC3 family members", VIROLOGY JOURNAL, BIOMED CENTRAL, LONDON, GB, vol. 3, no. 1, 19 October 2006 (2006-10-19), page 86, XP021025438, ISSN: 1743-422X, DOI: 10.1186/1743-422X-3-86
- C. TIBALDI ET AL: "Correlation of cytidine deaminase polymorphisms and activity with clinical outcome in gemcitabine-/platinum-treated advanced non-small-cell lung cancer patients", ANNALS OF ONCOLOGY., vol. 23, no. 3, 7 June 2011 (2011-06-07), pages 670-677, XP055451666, NL ISSN: 0923-7534, DOI: 10.1093/annonc/mdr280
- REBECCA KOHNKEN ET AL: "Regulation of deoxynucleotide metabolism in cancer: novel mechanisms and therapeutic implications", MOLECULAR CANCER, vol. 2, no. 5, 29 December 2015 (2015-12-29), page 401, XP055259382, GB ISSN: 1476-4598, DOI: 10.1186/s12943-015-0446-6 cited in the application

### SEQUENCE LISTING

<110> TRANSGENE SA
<120> Oncolytic viruses and therapeutic molecules
<130> AA3170 PCT S3
<150> EP 16 30 6831.5
   <151> 2016-12-28
<160> 3
<170> PatentIn version 3.5
<210> 1
   <211> 142
   <212> PRT
   <213> yeast
<400> 1
   Met Glu Val Gly Gly Ile Glu Asp Arg Gln Leu Glu Ala Leu Lys Arg
   1               5                   10                  15
   Ala Ala Leu Lys Ala Cys Glu Leu Ser Tyr Ser Pro Tyr Ser His Phe
               20                  25                  30
   Arg Val Gly Cys Ser Ile Leu Thr Asn Asn Asp Val Ile Phe Thr Gly
           35                  40                  45
   Ala Asn Val Glu Asn Ala Ser Tyr Ser Asn Cys Ile Cys Ala Glu Arg
       50                  55                  60
   Ser Ala Met Ile Gln Val Leu Met Ala Gly His Arg Ser Gly Trp Lys
   65                  70                  75                  80
   Cys Met Val Ile Cys Gly Asp Ser Glu Asp Gln Cys Val Ser Pro Cys
                   85                  90                  95
   Gly Val Cys Arg Gln Phe Ile Asn Glu Phe Val Val Lys Asp Phe Pro
               100                 105                 110
   Ile Val Met Leu Asn Ser Thr Gly Ser Arg Ser Lys Val Met Thr Met
           115                 120                 125
   Gly Glu Leu Leu Pro Met Ala Phe Gly Pro Ser His Leu Asn
       130                 135                 140
<210> 2
   <211> 146
   <212> PRT
   <213> human
<400> 2
<210> 3
   <211> 224
   <212> PRT
   <213> Homo sapiens
<400> 3
   Met Ala Gln Lys Glu Glu Ala Ala Val Ala Thr Glu Ala Ala Ser Gln
   1               5                   10                  15
   Asn Gly Glu Asp Leu Glu Asn Leu Asp Asp Pro Glu Lys Leu Lys Glu
               20                  25                  30
   Leu Ile Glu Leu Pro Pro Phe Glu Ile Val Thr Gly Glu Arg Leu Pro
           35                  40                  45
   Ala Asn Phe Phe Lys Phe Gln Phe Arg Asn Val Glu Tyr Ser Ser Gly
       50                  55                  60
   Arg Asn Lys Thr Phe Leu Cys Tyr Val Val Glu Ala Gln Gly Lys Gly
   65                  70                  75                  80
   Gly Gln Val Gln Ala Ser Arg Gly Tyr Leu Glu Asp Glu His Ala Ala
                   85                  90                  95
   Ala His Ala Glu Glu Ala Phe Phe Asn Thr Ile Leu Pro Ala Phe Asp
               100                 105                 110
   Pro Ala Leu Arg Tyr Asn Val Thr Trp Tyr Val Ser Ser Ser Pro Cys
           115                 120                 125
   Ala Ala Cys Ala Asp Arg Ile Ile Lys Thr Leu Ser Lys Thr Lys Asn
       130                 135                 140
   Leu Arg Leu Leu Ile Leu Val Gly Arg Leu Phe Met Trp Glu Glu Pro
   145                 150                 155                 160
   Glu Ile Gln Ala Ala Leu Lys Lys Leu Lys Glu Ala Gly Cys Lys Leu
                   165                 170                 175
   Arg Ile Met Lys Pro Gln Asp Phe Glu Tyr Val Trp Gln Asn Phe Val
               180                 185                 190
   Glu Gln Glu Glu Gly Glu Ser Lys Ala Phe Gln Pro Trp Glu Asp Ile
           195                 200                 205
   Gln Glu Asn Phe Leu Tyr Tyr Glu Glu Lys Leu Ala Asp Ile Leu Lys
       210                 215                 220

## Description

### TECHNICAL FIELD OF THE INVENTION

The present invention is in the field of oncolytic viruses and therapeutic genes. The invention provides oncolytic viruses comprising one or more therapeutic genes. More precisely, the therapeutic genes of the invention are nucleoside pool modulators. More particularly, the nucleoside pool modulators of the invention are cytidine deaminases or have a cytidine deaminase activity. The combination of the oncolytic viruses and one or more therapeutic genes as defined herein confers to the oncolytic viruses an increased antitumor efficacy. The invention finally provides oncolytic viruses for the prevention and/or the treatment of proliferative diseases. Any references to methods for treatment of the human or animal body by surgery or therapy and diagnostic methods practised on the human or animal body (see Art. 53(c) EPC) are to be reformulated in accordance with Article 54(4) or (5) EPC.

### BACKGROUND ART

Oncolytic viruses are a class of therapeutic agents that have the unique property of tumor-dependent self-perpetuation (Hermiston et al., 2006, Curr. Opin. Mol. Ther., 8(4):322-30). The benefit of using these viruses is that as they replicate, they lyse their host cells. Oncolytic viruses are capable of selective replication in dividing cells (e.g. cancer cells) while leaving non-dividing cells (e.g. normal cells) unharmed. As the infected dividing cells are destroyed by lysis, they release new infectious particles to infect the surrounding dividing cells. Therefore, oncolytic viruses offer a new area for treating cancer, optionally in association with conventional treatments for cancer (Fisher et al., 2006, Curr. Opin. Mol. Ther., 8(4):301-13). Cancer cells are ideal hosts for many viruses because they have the antiviral interferon pathway inactivated or have mutated tumour suppressor genes that enable viral replication to proceed unhindered (Chernajovsky et al., 2006, BMJ, 332(7534):170-2). Several viruses including adenovirus, reovirus, measles, herpes simplex, Newcastle disease virus and vaccinia virus have now been clinically tested as oncolytic agents.

Some viruses are naturally oncolytic and have an innate ability to selectively infect and kill tumor cells. However, oncolytic viruses can also be engineered by modifying naturally occurring viruses. For this purpose, the main strategies used currently to modify the viruses include: functional deletions in viral genes, the use of tumor- or tissue-specific promoters to control the expression of these viral genes, tropism modification to redirect virus to the cancer cell surface, among many other possibilities.

Among naturally oncolytic viruses, Vaccinia viruses (a Poxviridae) possess many of the key attributes necessary for an ideal viral backbone for use in oncolytic virotherapy. These include a short lifecycle, with rapid cell-to-cell spread, strong lytic ability, a large cloning capacity and well-defined molecular biology. In addition, although capable of replicating in human cells, they are not considered a natural health problem and are especially well characterized having been delivered to millions of individuals during the campaign to eradicate smallpox. Early clinical results using either vaccine strains or genetically modified vaccinia strains have demonstrated antitumor effects (Thorne et al., 2005, Curr. Opin. Mol. Ther., 7(4):359-65).

Viral modifications of the virus can be practiced in order to enhance the ability of viruses to infect and lyse 100% of the tumor cells which is difficult to achieve in in vivo context. Therefore, oncolytic viruses are often "armed" with enzyme-prodrug system which enhance the oncolytic efficacy of the virus therapy by exerting a strong bystander effect and thus permitting elimination of neighboring uninfected tumor cells. For example, armament with the so-called FCU1 suicide gene, encoding a bifunctional chimeric protein that combines the enzymatic activities of FCY1 and FUR1, efficiently catalyzed the direct conversion of 5-fluorocytosine (5-FC), a nontoxic antifungal agent, into the toxic metabolites 5-fluorouracil (5-FU) and 5-fluorouridine-5'monophosphate (5-FUMP), thus bypassing the natural resistance of certain human tumor cells to 5-fluorouracil (Erbs et al., 2000, Cancer Res., 60(14):3813-22).

Virus modifications can also be used to increase safety. In this regard, thymidine kinase (TK) deleted virus was shown to have decreased pathogenicity compared with wild type virus, but replication in tumor cells was preserved (Buller et al., 1985, Nature, 317(6040):813-5). Foloppe et al. showed that a TK gene-deleted VACV expressing the FCU1 gene has potent anti-tumor effect both *in vitro* and *in vivo* in a murine model of a human colon tumor (Foloppe et al., 2008, Gene Ther., 15:1361-71).

There is clearly an important need to develop effective approaches for the treatment of cancer. Attenuated oncolytic virus strains have been developed for therapeutic and diagnostic applications and are being evaluated in clinical studies. However, methods of attenuating oncolytic viruses lead to a decrease of their efficacy. In a therapeutic point of view, this decrease of efficacy can result into a diminution of overall response, a diminution of patient's survival, an increase of mortality, pathology resistances... As a result, there is a need of new safe oncolytic virus, which should be more efficient.

In the context of the present invention, the inventors have tested oncolytic viruses comprising one or more therapeutic genes. More particularly, therapeutic genes are nucleoside deaminases. Even more particularly, therapeutic genes are cytidine deaminase.

Cytidine Deaminase (CDAse) - also termed deoxycytidine deaminase or cytidine aminohydrolase - is a nucleoside pool modulator involved in the pyrimidine salvage pathway, and consequently involved in the balance of desoxyribonucleotides (dNTPs).

An appropriate balance of dNTPs is essential for the fidelity of DNA replication. In fact, it appears that the modulation of the dNTP pool balance has consequences on the DNA integrity, the genome stability, and can result in genotoxic damages. The two DNA strands of the nuclear genome are at similar risk of mutations resulting from this dNTP pool imbalance, and this risk is not completely suppressed even when both major replication error correction mechanisms are genetically intact. (Buckland et al., 2014 Dec, PLoS Genet., 10(12):e1004846). While a reduction or imbalance in dNTPs is known to result in genotoxicity and increased mutagenesis, an increase in dNTPs often results in uncontrolled DNA replication with reduced fidelity that can contribute to cancer development (Kohnken et al., 2015, Mol. Cancer; 14:176).

Therapeutic agents that target dNTP synthesis and metabolism can be used in treatment of several types of cancer. Despite several studies, the molecular mechanisms that regulate the intracellular dNTP levels and maintain their homeostasis are not completely understood. (Kohnken et al. 2015, Mol. Cancer; 14:176)

CDAse deficiency has been reported to lead to an excess of deoxycytidine triphosphate (dCTP). DNA replication in CDAse-deficient cells is unsuccessful due to the partial inhibition of basal poly-ADP-ribose polymerase 1 (PARP-1) activity. Indeed, the intracellular accumulation of dCTP inhibits PARP-1 activity compromising the activation of checkpoint kinase 1 and the downstream checkpoints efficiency, allowing the subsequent accumulation of unreplicated DNA in mitosis. This unreplicated DNA leads to the formation of ultrafine anaphase bridges (UFBs) between sister-chromatids at "difficult-to-replicate" sites such as centromeres and fragile sites. (Gemble et al., 2015, PLoS Genet, 11(7) e1005384)

CDAses are known to have an antiviral effect. APOBEC3G, a CDAse which deaminates complexed cytidines, is an innate defense protein showing activity against retroviruses and retrotransposons (Oliva et al., 2016, Immunol. Cell. Biol. 94:689-700). The role of APOBEC3 proteins in the inhibition of viral infection was firstly described for HIV-1. However, many studies have also shown evidence of APOBEC3 action on other viruses associated with human diseases, including HTLV, HCV, HBV, HPV, HSV-1, and EBV. APOBEC3G inhibits these viruses through a series of editing-dependent and independent mechanisms. Many viruses have evolved mechanisms to counteract APOBEC effects, and strategies that enhance APOBEC3 activity constitute a new approach for antiviral drug development. (Vieira et al., 2013, Biomed. Res. Int., 2013:ID 683095).

APOBEC has been reported to be a restriction factor for multiple DNA-containing viruses (Moris et al., 2014, Front Microbiol, 5:534). However, it has been shown that the APOBEC3 family members are not restriction factors for the replication of vaccinia virus, a poxvirus, and that vaccinia virus is not able to degrade APOBEC3G (Kremer et al., 2006, Virol. J., 3:86).

APOBEC gene has been reported to be overexpressed in non-small cell lung cancers. APOBEC3G may be the origin of clusters of mutation in various cancers, like breast cancer, lung cancer, head and neck cancer. The rate of mRNA APOBEC has been compared in cancerous and in normal lung tissues, and it appeared that mRNA APOBEC was significantly more elevated in cancerous tissues than in normal tissues (Hidefumi et al., 2014, Biomed. Rep., 2(3):392-395).

Moreover, a study was driven on the influence of the enzymatic activity of the CDAse on the clinical issue of NSCLC patients treated with platine-gemcitabine. It was reported that the enzymatic activity of CDAse, as detected by HPLC, was correlated with both activity and efficacy of platinum-gemcitabine therapy. More precisely, patients with a low-CDAse activity had a better response rate, a better clinical benefice, a better time to progression and a better overall survival than patients with a high-CDAse activity. Another preliminary study in 40 pancreatic cancer patients reported a similar correlation between high-CDAse activity and progression after gemcitabine-based treatments. The authors suggested the use of CDAse-enzymatic activity as a biomarker of activity and efficacy in patients treated with platinum-gemcitabine regimens (Tibaldi et al, 2012, Ann. Oncol., 23:670-677).

Some problems have been reported when cytidine deaminase or nucleoside pool modulators having a cytidine deaminase activity are used. With series of editing-dependent and independent mechanisms, APOBEC3 inhibits viruses, but also represents a source of diversification and evolution of the viral genetic. Many viruses developed new mechanisms to counteract APOBEC effects (Vieira et al., 2013, Biomed. Res. Int., 2013, Article ID 683095). Moreover, as mentioned above, CDAse can inhibit some cancer-treatment. WO2010118013 and WO2010118010 are directed to the combination of CDAse inhibitors and cytidine based antineoplastic drugs, like decitabine, gemcitabine, ara-C and 5-azacytidine. In fact, CDAse deaminates cytidine-based drugs, and consequently decreases their effects. These applications also relate to the use of these combinations for the treatment of cancers. In vivo efficacy study of a CDAse inhibitor (ER-876437) and gemcitabine in A2780 human ovarian cancer xenograft model showed that while ER-876437 alone and gemcitabine alone had no effect on the tumor growth, the administration of ER-876437 30 minutes prior to gemcitabine led to complete regression and partial tumor regression in 10% and 30% of the evaluated mice, respectively.

In the context of the present invention, the inventors have discovered, against all expectations, that oncolytic viruses engineered to express a cytidine deaminase have an improved efficacy. Anti-tumoral virotherapy comprising the administration of cytidine deaminase-expressing oncolytic vaccinia viruses results in a significant reduction of tumor progression. Moreover, contrary to other nucleoside pool modulators such as cytosine deaminase, the anti-tumor effect provided by the CDAse-expressing oncolytic virus does not require the addition of any prodrug, that also contributes to the ease of use and safety of the present invention.

Based on these results, one may anticipate that the oncolytic viruses of the invention may be successfully used as an alternative to the other existing oncolytic viruses, and may have a better efficiency profile. The oncolytic viruses of the invention can also be exploited in combination with additional anticancer therapy/ies.

### SUMMARY OF THE INVENTION

The present invention relates to the embodiments as characterized in the claims. Thus, it relates to the following items.
1. An oncolytic poxvirus defective in the J2R locus comprising a nucleotide sequence encoding a cytidine deaminase (CDAse) polypeptide, preferably selected from the group consisting of CDA, CDD, EC 3.5.4.5, APOBEC, AID and cytidine deaminase-like proteins.
2. The oncolytic poxvirus of item 1, wherein said APOBEC is selected from the group consisting of APOBEC1, APOBEC2, APOBEC3A, APOBEC3B, APOBEC3C, APOBEC3D, APOBEC3E, APOBEC3F, APOBEC3G, APOBEC3H and APOBEC4, and preferably wherein said APOBEC is APOBEC2.
3. The oncolytic poxvirus of item 1, wherein said CDAse is a yeast cytidine deaminase (CDD1), preferably a CDD1 comprising an amino acid sequence having at least 80%, preferably greater than 90%, and more preferably greater than 95% identity with SEQ ID NO:1, or a human cytidine deaminase (hCD), preferably a hCD comprising an amino acid sequence having at least 80%, preferably greater than 90%, and more preferably greater than 95% identity with SEQ ID NO:2.
4. The oncolytic poxvirus of item 2, wherein said APOBEC2 comprises an amino acid sequence having at least 80%, preferably greater than 90%, and more preferably greater than 95% identity with SEQ ID NO:3.
5. The oncolytic poxvirus of any of items 1 to 4, wherein said CDAse polypeptide catalyses the deamination of cytidine-based components, preferably cytidine-based components that are free or complexed, to uridine-based components, like cytidine to uridine and deoxycytidine (dC) to deoxyuridine (dU).
6. The oncolytic poxvirus of item 1, wherein said poxvirus is a poxvirus belonging to the Orthopoxvirus genus, wherein said poxvirus preferably belongs to the vaccinia virus (VV) species, preferably selected from the group consisting of Copenhagen, Wyeth and Western Reserve (WR), or to the cowpox virus species.
7. The oncolytic poxvirus of item 1, wherein said virus is further defective in the I4L and/or F4L locus.
8. The oncolytic poxvirus of item 7, wherein said poxvirus is a vaccinia virus defective in viral TK and RR encoded activities which encodes a human or yeast CDAse.
9. The oncolytic poxvirus of any of items 6 to 8, wherein said virus further comprises one or more nucleic acid(s) of interest, said nucleic acid of interest preferably encoding an immunostimulatory polypeptide, an antigen or a permease.
10. The oncolytic poxvirus of any of items 1 to 9, wherein said virus further comprises the elements necessary for the expression of said cytidine deaminase and said one or more nucleic acid(s) of interest.
11. A process for preparing an oncolytic poxvirus, in which process:
   (i) An oncolytic poxvirus according to anyone of items 1 to 10 is introduced into a producer cell;
   (ii) said producer cell is cultured under conditions which are appropriate for enabling said oncolytic poxvirus to be produced, and;
   (iii) said oncolytic poxvirusis recovered from the cell culture.
12. A composition comprising the oncolytic poxvirus of any one of items 1 to 10, or prepared according to item 11, which further comprises a pharmaceutically acceptable vehicle, wherein said composition preferably comprises a dose of oncolytic virus comprised between 10⁶ and 5×10⁹ PFU, and wherein said oncolytic virus is preferably formulated for parenteral route administration with a preference for intravenous or intratumoral route.
13. The oncolytic poxvirus of any one of items 1 to 10, or prepared according to item 11, or the composition of item 12, for use in the treatment of a disease or a pathologic condition in a subject in need thereof.
14. The oncolytic poxvirus of any one of items 1 to 10, or prepared according to item 11, or the composition of item 12, for use in the prophylaxis or the treatment of a proliferative disease, said proliferative disease preferably being selected from the group consisting of cancer, tumor and restenosis, wherein said cancer is preferably a gastro-intestinal cancer, preferably comprising a cancer of oesophagus, gallbladder, liver, pancreas, stomach, small intestine, bowel (large intestine or colon and rectum), or anus.
15. The oncolytic poxvirus for use according to item 14, further comprising the administration of one or more substances effective in anticancer therapy.

Thus, one aspect of the invention relates to an oncolytic poxvirus as defined above comprising a nucleotide sequence encoding a cytidine deaminase (CDAse) polypeptide.

In one embodiment, the oncolytic poxvirus as defined above of the invention is a poxvirus belonging to the Orthopoxvirus and especially a vaccinia virus or a cowpoxvirus.

The oncolytic poxvirus as defined above of the invention is defective in the thymidine kinase (TK-) encoding J2R locus.

In yet another embodiment, the oncolytic poxvirus as defined above of the invention is defective in the

I4L and/or F4L locus (alternatively or in combination with a defective TK- locus).

In a further embodiment, said oncolytic poxvirus as defined above further comprises a nucleic acid of
interest, in particular a gene encoding a therapeutic molecule different from CDAse, such as an immunostimulatory polypeptide, an antigen, a permease or any other molecules of interest.

In another aspect, the present invention further provides a composition comprising the oncolytic poxvirus as defined above of the present invention an a pharmaceutically acceptable vehicle. In
one embodiment, the oncolytic virus is preferably formulated for intra-venous or intratumoral administration.

In a further aspect, the present invention also concerns a process for preparing the oncolytic poxvirus as defined above, comprising at least the steps of introducing said oncolytic virus into a
producer cell, culturing the producer cell under conditions that are appropriate for enabling said oncolytic virus to be produced and recovering the produced virus from the cell culture. Optionally, the recovered oncolytic virus can be purified at least partially.

In still a further aspect, the present invention provides the oncolytic poxvirus as defined above or the
composition of the invention, for use for the prophylaxis and/or the treatment of a disease. In one embodiment, said disease is a proliferative disease such as cancers, tumors and restenosis. Said cancer is preferably selected from the group consisting in renal cancer, bladder cancer, prostate cancer, breast cancer, colorectal cancer, lung cancer, hepatic cancer, gastric cancer, pancreatic cancer, melanoma, ovarian cancer and glioblastoma, and especially metastatic ones. In another embodiment, said disease is a disease associated with an increased osteoclast activity like rheumatoid arthritis and osteoporosis.

There is also disclosed a method of treating a disease which comprises the administration into a host organism in need thereof of a therapeutically effective amount of the oncolytic virus or the composition of the invention. Said method of treatment may be used in conjunction with one or more additional therapies such as ones selected from the group consisting of surgery, radiotherapy, chemotherapy, cryotherapy, hormonal therapy, toxin therapy, immunotherapy or cytokine therapy.

As disclosed herein, cytidine deaminase is used as a therapeutic gene, and not as a suicide gene, insofar as no prodrug is required to obtain a therapeutic effect in the presence of cytidine deaminase. Specifically, capecitabine (Xeloda^{®}) is transformed into 5'-DFCR (Deoxy-5-fluorocytidine), which is then transformed by cytidine deaminase into 5'-DFUR (5'-Deoxy-5-fluorouridine), which is finally transformed into toxic 5-FU (5-fluoro-uracile). This cascade of reaction is not required to obtain cytolytic activity of the oncolytic poxvirus as defined above of the present invention. Accordingly, the oncolytic poxvirus as defined above of the present invention is
not used in combination with capecitabine and the method of treatment according to the present invention does not comprise any step of administering a precursor of a drug such as capecitabine. Usually, nucleoside pool modulators other than CDAse are used in combination with a prodrug which is transformed in a cytotoxic drug by the action of said nucleoside pool modulators. Such an enzymatic transformation generally contributes to the anti-tumor effect. For example, a method of treating relying on an oncolytic virus encoding a cytosine deaminase provides an effective anti-tumor effect when administered in conjunction with the 5-fluorocytosine (5-FC) which is transformed in cytotoxic 5-fluorouracile (5-FU) in the infected cells and contributes to their lysis.

### DESCRIPTION OF THE FIGURES

**Figure 1****. Specific detection of the CDA (cytidine deaminase) protein on Western blots by antibody against human CDA (hCD).**

MIA PaCa-2 and LoVo cells were infected with VVTK-RR- and VV-TK-RR-/hCD. The presence of hCD (16 kDa) is indicated (arrow).

**Figure 2****. Growth suppression of human colorectal LoVo tumors implanted s.c. after one i.v. injection of VVTK-RR-/GFP or VVTK-RR-/CDD1.**

**Abbreviations: GFP: green fluorescent protein; CDD1: yeast cytidine deaminase**

Mean tumor volume after systemic treatment with 1 × 10⁷ PFU of oncolytic vaccinia virus (indicated by arrow). Mice bearing LoVo s.c. xenografts were treated with one i.v. administration (in tail vein) at day 11 with vehicle alone (◇: control) or with a TK and RR-defective virus expressing the green fluorescent protein (GFP) (□: VVTK-RR-/GFP) or expressing the yeast CDAse (■: VVTK-RR-/CDD1). The data represent the mean of 12 animals.

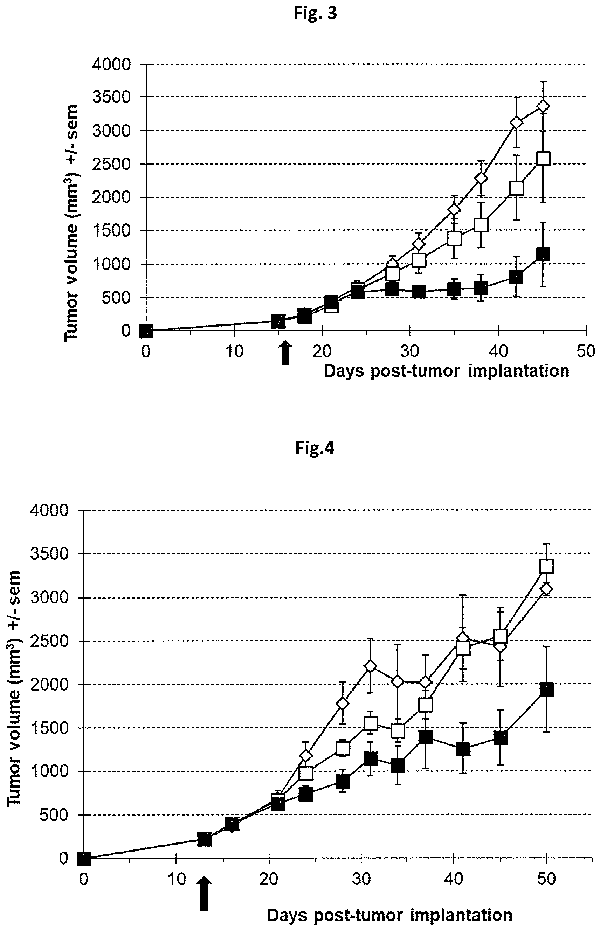

**Figure 3****.Growth suppression of human colorectal HCT-116 tumors implanted s.c. after one i.v. injection of VVTK-RR-/GFP or VVTK-RR-/CDD1.**

Mean tumor volume after systemic treatment with 1 × 10⁵ PFU of oncolytic vaccinia virus (indicated by arrow). Mice bearing HCT-116 s.c. xenografts were treated with one i.v. administration (in tail vein) at day 16 with vehicle alone (◇: control) or with a TK and RR defective virus expressing GFP (□: VVTK-RR-/GFP) or expressing the yeast CDAse (■: VVTK-RR-/CDD1). The data represent the mean of 12 animals.

**Figure 4****. Growth suppression of human esophageal OE-19 tumors implanted s.c. after one i.v. injection of VVTK-RR-/GFP or VVTK-RR-/CDD1.**

Mean tumor volume after systemic treatment with 1 × 10⁶ PFU of oncolytic vaccinia virus (indicated by arrow). Mice bearing OE-19 s.c. xenografts were treated with one i.v. administration (in tail vein) at day 13 with vehicle alone (◇: control) or with a TK and RR defective virus expressing GFP (□: VVTK-RR-/GFP) or expressing the yeast CDAse (■: VVTK-RR-/CDD1). The data represent the mean of 12 animals.

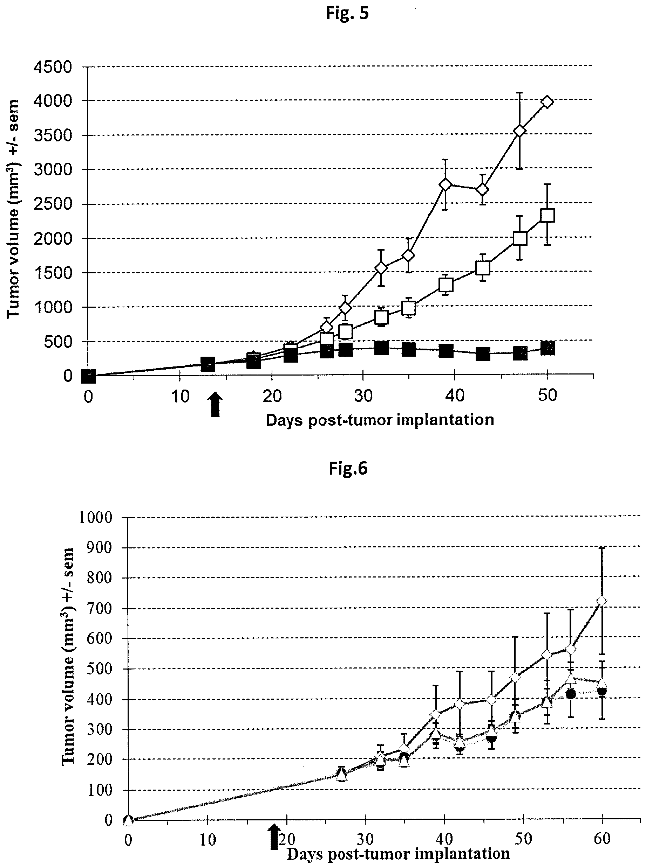

**Figure 5****. Growth suppression of human esophageal OE-19 tumors implanted s.c. after one i.v. injection of VVTK-RR-/GFP or VVTK-RR-/hCD.**

Mean tumor volume after systemic treatment with 5 × 10⁵ PFU of oncolytic vaccinia virus (indicated by arrow). Mice bearing OE-19 s.c. xenografts were treated with one i.v. administration (by tail vein) at day 14 with vehicle alone (◇: control) or with a TK and RR defective virus expressing GFP (□: VVTK-RR-/GFP) or expressing the human CDAse (■: VVTK-RR-/hCD). The data represent the mean of 12 animals.

**Figure 6****. Growth suppression of human colorectal LoVo tumors implanted s.c. after one i.v. injection of VVTK-RR- or VVTK-RR-/FCU1**

Mean tumor volume after systemic treatment with 1 × 10⁷ PFU of oncolytic vaccinia virus (indicated by arrow). Mice bearing LoVo s.c. xenografts were treated with one i.v. administration (by tail vein) at day 27 with vehicle alone (◇: control) or with a non-recombinant TK and RR defective virus (•: VVTK-RR-) or a TK and RR defective expressing yeast cytosine deaminase and UPRTase (Δ: VVTK-RR-FCU1). The data represent the mean of 12 animals.

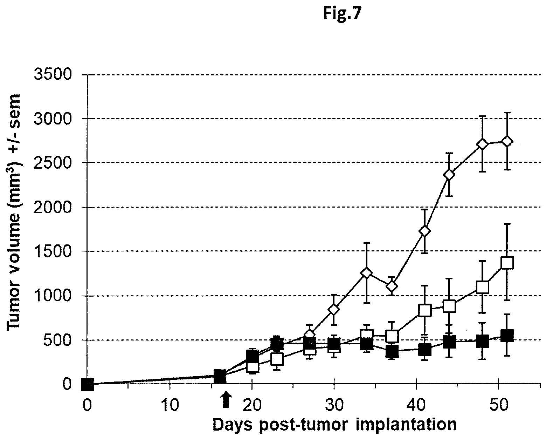

**Figure 7****. Growth suppression of human colorectal HCT-116 tumors implanted s.c. after one i.v. injection of VVTK-RR-/GFP or VVTK-RR-/APOBEC2.**

Mean tumor volume after systemic treatment with 1 × 10⁵ PFU of replicating vaccinia virus (indicated by arrow). Mice bearing HCT-116 s.c. xenografts were treated with one i.v. administration (by tail vein) at day 17 with vehicle alone (◇: control) or with the indicated virus (□: VVTK-RR-/GFP; ■: VVTK-RR-/APOBEC2). The data represent the mean of 9 animals.

### DETAILED DESCRIPTION OF THE INVENTION

### Definitions

As used throughout the entire application, the terms "a" and "an" are used in the sense that they mean "at least one", "at least a first", "one or more" or "a plurality" of the referenced components or steps, unless the context clearly dictates otherwise. For example, the term "an oncolytic virus" includes a plurality of oncolytic viruses, including mixtures thereof.

The term "one or more" refers to either one or a number above one (e.g. 2, 3, 4, etc.).

The term "and/or" wherever used herein includes the meaning of "and", "or" and "all or any other combination of the elements connected by said term".

The term "about" or "approximately" as used herein means within 20%, preferably within 10%, and more preferably within 5% of a given value or range.

As used herein, when used to define products and compositions, the terms "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are open-ended and do not exclude additional, un-recited elements or method steps. The expression "consisting essentially of" means excluding other components or steps of any essential significance. Thus, a composition consisting essentially of the recited components would not exclude traces, contaminants and pharmaceutically acceptable carriers. "Consisting of" shall mean excluding more than trace elements of other components or steps.

The terms "polypeptide", "peptide" and "protein" refer to polymers of amino acid residues which comprise at least nine or more amino acids bonded via peptide bonds. The polymer can be linear, branched or cyclic and may comprise naturally occurring and/or amino acid analogues and it may be interrupted by non-amino acids. As a general indication, if the amino acid polymer is more than 50 amino acid residues, it is preferably referred to as a polypeptide or a protein whereas if it is 50 amino acids long or less, it is referred to as a "peptide".

Within the context of the present invention, the terms "nucleic acid", "nucleic acid molecule", "polynucleotide" and "nucleotide sequence" are used interchangeably and define a polymer of any length of either polydeoxyribonucleotides (DNA) (e.g. cDNA, genomic DNA, plasmids, vectors, viral genomes, isolated DNA, probes, primers and any mixture thereof) or polyribonucleotides (RNA) (e.g. mRNA, antisense RNA, SiRNA) or mixed polyribo-polydeoxyribonucleotides. They encompass single or double-stranded, linear or circular, natural or synthetic, modified or unmodified polynucleotides. Moreover, a polynucleotide may comprise non-naturally occurring nucleotides and may be interrupted by non-nucleotide components.

The term "analogue", "mutant" or "variant" as used herein refers to a molecule (polypeptide or nucleic acid) exhibiting one or more modification(s) with respect to the native counterpart. Any modification(s) can be envisaged, including substitution, insertion and/or deletion of one or more nucleotide/amino acid residue(s). When several mutations are contemplated, they can concern consecutive residues and/or non-consecutive residues. Preferred are analogues that retain a degree of sequence identity of at least 80%, advantageously at least 85%, preferably at least 90%, more preferably at least 95%, and even more preferably at least 98% identity with the sequence of the native counterpart. For illustrative purposes, "at least 80% identity" means 80%, 81%, 82%, 83%, 84%, 85%, 86%, 87%, 88%, 89%, 90%, 91%, 92%, 93%, 94%, 95%, 96%, 97%, 98%, 99% or 100%. In a general manner, the term "identity" refers to an amino acid to amino acid, or nucleotide to nucleotide correspondence between two polypeptides or nucleic acid sequences. The percentage of identity between two sequences is a function of the number of identical positions shared by the sequences, taking into account the number of gaps which need to be introduced for optimal global alignment and the length of each gap. Various computer programs and mathematical algorithms are available in the art to determine the percentage of identity between amino acid sequences after global alignment, such as for example the algorithm of Needleman et Wunsh, J.Mol. Biol. 48,443-453, 1970, and the Blast program available at NCBI or ALIGN in Atlas of Protein Sequence and Structure (Dayhoffed, 1981, Suppl., 3: 482-9). Programs for determining identity between nucleotide sequences are also available in specialized data base (e.g. Genbank, the Wisconsin Sequence Analysis Package, BESTFIT, FASTA and GAP programs, and the needle software available from ebi.ac.uk worldwide under the name « Align »).

As used herein, the term "host cell" should be understood broadly without any limitation concerning particular organization in tissue, organ, or isolated cells. Such cells may be of a unique type of cells or a group of different types of cells such as cultured cell lines, primary cells and dividing cells. In the context of the invention, the term "host cells" include prokaryotic cells, lower eukaryotic cells such as yeast, and other eukaryotic cells such as insect cells, plant and mammalian (e.g. human or non-human) cells as well as cells capable of producing the oncolytic virus of the invention. This term also includes cells which can be or has been the recipient of the virus described herein as well as progeny of such cells.

The terms "virus", 'viral particle", "viral vector" and virion" are used interchangeably and are to be understood broadly as meaning a vehicle comprising at least one element of a wild-type virus genome and may be packaged into a viral particle or to a viral particle. Although, viral particles may or may not contain nucleic acid (i.e. the viral genome) it is preferred that a virus comprises a DNA or RNA viral genome packaged into a viral particle (or virion) and is infectious (i.e. capable of infecting and entering a host cell or subject). Desirably, the virus of this invention is associated with a DNA genome, and most preferably a double-stranded DNA genome. In the context of the present disclosure, a "virus" includes wild-type and engineered viruses.

The term "naturally occurring" or "wild-type" or "native" is used to describe a biological molecule or organism that can be found in nature as distinct from being artificially produced by man. For example, a virus which can be isolated from a source in nature is wild-type. The present invention also encompasses wild-type viruses that can be obtained from specific collections (e.g. ECCAC, ATCC, CNCM, etc.). A biological molecule or an organism which has been intentionally modified by man in the laboratory is not naturally occurring. Representative examples of non-naturally occurring viruses include, among many others, recombinant viruses engineered by insertion of one or more gene(s) of interest in the viral genome and mutated viruses engineered by total or partial deletion of a viral gene to make the modified virus defective for the encoded gene product (e.g. tk- virus) as well as chimeric viruses containing genomic fragments obtained from different virus origins.

The term "obtained from", "originating" or "originate" is used to identify the original source of a component (e.g. polypeptide, nucleic acid molecule, virus, etc.) but is not meant to limit the method by which the component is made which can be, for example, by chemical synthesis or recombinant means.

As used herein, the term "oncolytic virus" refers to any virus, naturally occurring, engineered or otherwise modified, which is capable of selectively replicating in dividing cells (e.g. a proliferative cell such as a cancer cell) with the aim of slowing the growth and/or lysing said dividing cell, either in vitro or in vivo, while showing no or minimal replication in non-dividing cells (e.g. primary cells).

The terms "replication" and "propagation" are used interchangeably and refer to the ability of a virus to reproduce and proliferate. Virus replication can be quantified at the level of nucleic acid or at the level of infectious viral particle using assays standard in the art and described herein such as a virus titer assay, plaque assay, absorbance, fluorescence detection, mass spectrometry, etc.

The term "therapeutic gene" as used herein refers to a nucleic acid sequence encoding a product, also called a "therapeutic molecule", capable of providing a biological activity when administered appropriately to a subject, especially to a patient suffering from a disease or illness condition or who should be protected against this disease or condition. This biological activity is expected to cause a beneficial effect on the course or a symptom of the pathological condition to be treated by either potentiating anti-tumor efficacy or reinforcing the oncolytic nature of the virus.

The term "treatment" (and any form of treatment such as "treating", "treat") as used herein encompasses prophylaxis (e.g. preventive measure in a subject at risk of having the pathological condition to be treated) and/or therapy (e.g. in a subject diagnosed as having the pathological condition), optionally in association with conventional therapeutic modalities. The result of the treatment is to slow down, cure, ameliorate or control the progression of the targeted pathological condition. For example, a subject is successfully treated for a cancer if after administration of an oncolytic virus as described herein, alone or in combination, the subject shows an observable improvement of its clinical status.

The term "administering" (or any form of administration, such as "administered") as used herein refers to the delivery to a subject of a therapeutic agent such as the oncolytic virus described herein.

As used herein, the term "proliferative disease" encompasses any disease or condition resulting from uncontrolled cell growth and spread including cancers, tumors and some cardiovascular diseases (restenosis that results from the proliferation of the smooth muscle cells of the blood vessel wall, etc.). The term "cancer" may be used interchangeably with any of the terms "tumor", "malignancy", "neoplasm", etc. These terms are meant to include any type of tissue, organ or cell, any stage of malignancy (e.g. from a pre-lesion to stage IV).

As used herein, the term "disease associated with an increased osteoclast activity" encompasses any disease or condition resulting in bone resorption or destruction (e.g. rheumatoid arthritis, osteoporosis, etc.).

The term "subject" generally refers to an organism for whom any product and method of the invention is needed or may be beneficial. Typically, the organism is a mammal, particularly a mammal selected from the group consisting of domestic animals, farm animals, sport animals, and primates. Preferably, the subject is a human who has been diagnosed as having or at risk of having a proliferative disease such as a cancer. The terms "subject" and "patients" may be used interchangeably when referring to a human organism and encompasses male and female. The subject to be treated may be a new-born, an infant, a young adult, an adult or an elderly.

The terms "combination treatment", "combination therapy", "combined treatment" or "combinatorial treatment", may be used interchangeably and refer to a treatment of a subject with at least two different therapeutic agents. In one embodiment, one of the therapeutic agent is said oncolytic virus. The second therapeutic agent may be any clinically established therapeutic agent, in particular one selected from the group consisting of surgery, radiotherapy, chemotherapy, cryotherapy, hormonal therapy, toxin therapy, immunotherapy, cytokine therapy, targeted cancer therapy, gene therapy, photodynamic therapy, transplantation etc. A combinatorial treatment may include a third or even further therapeutic agent. For combination treatment, it is appreciated that optimal concentration of each component of the combination can be determined by the artisan skilled in the art. In a specific embodiment, the oncolytic virus of the invention (e.g. wild type oncolytic virus, or a modified derivative oncolytic virus) may be delivered in combination with cytidine deaminase, wherein said cytidine deaminase is not encoded by the oncolytic virus. Cytidine deaminase may be in the form of a polypeptide (e.g. a recombinantly produced cytidine deaminase, or analogue thereof) or of a nucleic acid molecule (e.g. carried by a vector engineered for expressing such cytidine deaminase(s)), as well as mixture of polypeptide(s) and nucleic acid molecule(s) (e.g. cytidine deaminase(s) and expressing vector(s)).

### Oncolytic viruses

The "oncolytic virus" as disclosed in the context of the present invention can be obtained from any member of
virus identified at present time provided that it is oncolytic by a higher propensity to replicate in and kill dividing cells as compared to non-dividing cells. It may be a native virus that is naturally oncolytic or may be engineered by modifying one or more viral genes so-as to increase tumor selectivity and/or preferential replication in dividing cells, such as those involved in DNA replication, nucleic acid metabolism, host tropism, surface attachment, virulence, lysis and spread (see for example Kirn et al., 2001, Nat. Med. 7: 781; Wong et al., 2010, Viruses 2: 78-106). One may also envisage placing one or more viral gene(s) under the control of event or tissue-specific regulatory elements (e.g. promoter).

Exemplary oncolytic viruses as disclosed herein include without limitation reovirus, Seneca Valley Virus
(SVV), vesicular stomatitis virus (VSV), Newcastle disease virus (NDV), herpes simplex virus (HSV), morbillivirus, adenovirus, poxvirus, retrovirus, measles virus, foamy virus, alpha virus, lentivirus, influenza virus, Sinbis virus, myxoma virus, rhabdovirus, picornavirus, coxsackievirus, parvovirus or the like.

The oncolytic virus as disclosed herein may be obtained from a
reovirus. A representative example includes Reolysin (under development by Oncolytics Biotech; NCT01166542).

The oncolytic virus as disclosed herein may be obtained from a
Seneca Valley virus. A representative example includes NTX-010 (Rudin et al., 2011, Clin. Cancer. Res. 17(4): 888-95).

The oncolytic virus as disclosed herein may be obtained from a
vesicular stomatitis virus (VSV). Representative examples are described in the literature (e.g. Stojdl et al., 2000, Nat. Med. 6(7): 821-5; Stojdl et al., 2003, Cancer Cell 4(4): 263-75).

The oncolytic virus as disclosed herein may be obtained from a
Newcastle disease virus. Representative examples include without limitation the 73-T PV701 and HDV-HUJ strains as well as those described in the literature (e.g. Phuangsab et al., 2001, Cancer Lett. 172(1):27-36; Lorence et al., 2007, Curr. Cancer Drug Targets 7(2):157-67; Freeman et al., 2006, Mol. Ther. 13(1):221-8).

The oncolytic virus as disclosed herein may be obtained from a
herpes virus. The Herpesviridae are a large family of DNA viruses that all share a common structure and are composed of relatively large double-stranded, linear DNA genomes encoding 100-200 genes encapsided within an icosahedral capsid which is enveloped in a lipid bilayer membrane. Although the oncolytic herpes virus can be derived from different types of HSV, particularly preferred are HSV1 and HSV2. The herpes virus may be genetically modified so-as to restrict viral replication in tumors or reduce its cytotoxicity in non-dividing cells. For example, any viral gene involved in nucleic acid metabolism may be inactivated, such as thymidine kinase (Martuza et al., 1991, Science 252: 854-6), ribonucleotide reductase (RR) (Boviatsis et al., Gene Ther. 1: 323-31; Mineta et al., 1994, Cancer Res. 54: 3363-66), or uracil-N-glycosylase (Pyles et al., 1994, J. Virol. 68: 4963-72). Another aspect involves viral mutants with defects in the function of genes encoding virulence factors such as the ICP34.5 gene (Chambers et al., 1995, Proc. Natl. Acad. Sci. USA 92: 1411-5). Representative examples of oncolytic herpes virus include NV1020 (e.g. Geevarghese et al., 2010, Hum. Gene Ther. 21(9): 1119-28) and T-VEC (Harrington et al., 2015, Expert Rev. Anticancer Ther. 15(12):1389-1403).

The oncolytic virus as disclosed herein may be obtained from a
morbillivirus which can be obtained from the paramyxoviridae family, with a specific preference for measles virus. Representative examples of oncolytic measles viruses include without limitation MV-Edm (McDonald et al., 2006; Breast Cancer Treat. 99(2): 177-84) and HMWMAA (Kaufmann et al., 2013, J. Invest. Dermatol. 133(4): 1034-42)

The oncolytic virus as disclosed herein may be obtained from an
adenovirus. Methods are available in the art to engineer oncolytic adenoviruses. An advantageous strategy includes the replacement of viral promoters with tumor-selective promoters or modifications of the E1 adenoviral gene product(s) to inactivate its/their binding function with p53 or retinoblastoma (Rb) protein that are altered in tumor cells. In the natural context, the adenovirus E1B55kDa gene cooperates with another adenoviral product to inactivate p53 (p53 is frequently dysregulated in cancer cells), thus preventing apoptosis. Representative examples of oncolytic adenovirus include ONYX-015 (e.g. Khuri et al., 2000, Nat. Med 6(8): 879-85) and H101 also named Oncorine (Xia et al., 2004, Ai Zheng 23(12): 1666-70).

The oncolytic virus of the present invention is a poxvirus. As used herein the term "poxvirus" or "poxviral vector" refers to a virus belonging to the Poxviridae family, with a specific preference for a poxvirus belonging to the Chordopoxviridae subfamily and more preferably to the Orthopoxvirus genus. Sequences of the genome of various poxviruses, for example, the vaccinia virus, cowpox virus, canarypox virus, ectromelia virus, myxoma virus genomes are available in the art and specialized databases such as Genbank (accession number NC_006998, NC_003663 or AF482758.2, NC_005309, NC_004105, NC_001132 respectively).

Advantageously, the oncolytic poxvirus is an oncolytic cowpox virus, and can derive from any cowpox strain, like for example CPXV_GER1980_EP4 (Genbank HQ420895), CPXV_GER2002_MKY (Genbank HQ420898), CPXV_GER1991_3 (Genbank DQ 437593), CPXV_FRA2001_NANCY (Genbank HQ420894), CPXV_GER1990_2 (Genbank HQ420896), CPXV_UK2000_K2984 (Genbank HQ420900), CPXV_BR (Genbank AF482758.2 or NC 003663) and CPXV_NOR1994-MAN (Genbank HQ420899), CPXV_GER1998_2 (Genbank HQ420897), CPXV_gri (Genbank X94355), CPXV_FIN2000_MAN (Genbank HQ420893) and CPXV_AUS1999_867 (Genbank HQ407377).

Desirably, the oncolytic poxvirus is an oncolytic vaccinia virus. Vaccinia viruses are members of the poxvirus family characterized by a 200kb double-stranded DNA genome that encodes numerous viral enzymes and factors that enable the virus to replicate independently from the host cell machinery. The majority of vaccinia virus particles is intracellular (IMV for intracellular mature virion) with a single lipid envelop and remains in the cytosol of infected cells until lysis. The other infectious form is a double enveloped particle (EEV for extracellular enveloped virion) that buds out from the infected cell without lysing it.

Although it can derive from any vaccinia virus strain, Elstree, Wyeth, Copenhagen and Western Reserve strains are particularly preferred. The gene nomenclature used herein is that of Copenhagen vaccinia strain. It is also used herein for the homologous genes of other poxviridae unless otherwise indicated. However, gene nomenclature may be different according to the pox strain but correspondence between Copenhagen and other vaccinia strains are generally available in the literature.

Preferably, the oncolytic vaccinia virus of the present invention is modified by altering one or more viral gene(s). Said modification(s) preferably lead(s) to the synthesis of a defective protein unable to ensure the activity of the protein produced under normal conditions by the unmodified gene (or lack of synthesis). Modifications encompass deletion, mutation and/or substitution of one or more nucleotide(s) (contiguous or not) within the viral gene or its regulatory elements. Modification(s) can be made in a number of ways known to those skilled in the art using conventional recombinant techniques. Exemplary modifications are disclosed in the literature with a specific preference for those altering viral genes involved in DNA metabolism, host virulence, IFN pathway (see e.g. Guse et al., 2011, Expert Opinion Biol. Ther.11(5): 595-608) and the like.

The oncolytic poxvirus as defined above of the present invention is modified by altering the thymidine kinase-encoding gene (locus J2R). The TK enzyme is involved in the synthesis of deoxyribonucleotides. TK is needed for viral replication in normal cells as these cells have generally low concentration of nucleotides whereas it is dispensable in dividing cells which contain high nucleotide concentration.

Alternatively, or in combination, the oncolytic poxvirus as defined above of the present invention is
modified by altering at least one gene or both genes encoding ribonucleotide reductase (RR). In the natural context, this enzyme catalyzes the reduction of ribonucleotides to deoxyribonucleotides that represents a crucial step in DNA biosynthesis. The viral enzyme is similar in subunit structure to the mammalian enzyme, being composed of two heterologous subunits, designed R1 and R2 encoded respectively by the I4L and F4L locus. Sequences for the I4L and F4L genes and their locations in the genome of various poxvirus are available in public databases, for example via accession number DQ437594, DQ437593, DQ377804, AH015635, AY313847, AY313848, NC_003391, AF482758.2, NC_003389, NC_003310, AY243312, DQ011157, DQ011156, DQ011155, DQ011154, DQ011153, Y16780, AF438165, U60315, AF410153, AF380138, L22579, NC_006998, DQ121394 and NC_008291. In the context of the invention, either the I4L gene (encoding the R1 large subunit) or the F4L gene (encoding the R2 small subunit) or both may be inactivated.

Alternatively, or in combination, other strategies may also be pursued to further increase the virus tumor-specificity. A representative example of suitable modification includes disruption of the VGF-encoding gene from the viral genome. VGF (for W growth factor) is a secreted protein which is expressed early after cell infection and its function seems important for virus spread in normal cells. Another example is the disruption of the A56R gene coding for hemagglutinin, optionally in combination with TK deletion (Zhang et al., 2007, Cancer Res. 67:10038-46). Disruption of interferon modulating gene(s) may also be advantageous (e.g. the B8R or B18R gene) or the caspase-1 inhibitor B13R gene. Another suitable modification comprises the disruption of the F2L gene which encodes the viral dUTPase involved in both maintaining the fidelity of DNA replication and providing the precursor for the production of TMP by thymidylate synthase (Broyles et al., 1993, Virol. 195: 863-5). Sequence of the vaccinia virus F2L gene is available in Genbank via accession number M25392 and a poxvirus defective in F2L locus is available from WO2009/065547.

The oncolytic virus of this invention is a poxvirus defective in the J2R locus, resulting from inactivating mutations, so-as to lack viral TK activity. In another preferred embodiment, the oncolytic virus of this invention is a poxvirus defective in both TK and RR activities resulting from inactivating mutations in both the J2R locus and at least one of the RR-encoding I4L and/or F4L locus carried by the viral genome (e.g. as described in WO2009/065546 and Foloppe et al., 2008, Gene Ther., 15: 1361-71).

In another embodiment, the oncolytic virus of this invention is a poxvirus defective for dUTPase resulting from inactivating mutations in the F2L gene (e.g. as described in WO2009/065547), optionally in combination with disruption of at least one of TK and RR activities or both (resulting in a virus with inactivating mutations in the F2L; F2L and J2R gene; F2L and I4L; or F2L, J2R and I4L).

### Nucleoside pool modulator and cytidine deaminase

The term "nucleoside" refers to any of various compounds consisting of a sugar, usually ribose or deoxyribose, and a purine or pyrimidine base. More precisely, the term "ribonucleoside" designates any purine or pyrimidine base linked to a ribose. Similarly, the term "deoxyribonucleoside" refers to any purine or pyrimidine base linked to a deoxyribose. As a result, the expression "nucleoside" designates both ribonucleosides and deoxyribonucleosides. Examples of nucleoside is cytidine and deoxycytidine.

The term "nucleotide" refers to any of various compounds consisting of a nucleoside and one or more phosphates. The expression "nucleotide" designates both ribonucleotides and deoxyribonucleotides. Examples of nucleotides are cytidine-monophosphate, cytidine-diphosphate or cytidine tri-phosphate.

As used herein, the term "nucleoside pool modulator" or "modulator of nucleoside pool" refers to any component which directly or indirectly acts on the nucleoside pool, in a negative or a positive way. Similarly, the term "nucleotide pool modulator" or "modulator of nucleotide pool" refers to any component which directly or indirectly acts on the nucleotide pool in a negative or a positive way. The term "modulator" stands for entire modulator(s), parts (e.g. truncated) and analogues thereof. The term "modulators" encompasses "cellular" modulators which are naturally present in a host cell, such as in humans, animals, insects or microorganisms (e.g. virus, bacteria). The expression of cellular nucleoside pool modulators may be increased in dividing cells (De Pas et al., 2008, J. Clin. Oncol., 20;26:14644). In the context of the present invention, such "modulator(s)" include modulators that increase the pool of nucleos/tides as well as those that decrease the pool of nucleos/tides. Such modulator(s) may directly or indirectly act on the pool of nucleosides or nucleotides. The nucleosides and nucleotides of the invention can whether be endogenous (e.g. de novo biosynthesis, salvage pathway) or exogenous (e.g. degradation of food or drugs). They also can be free (e.g. in the cytoplasm) or complexed (e.g. in the DNA). In one embodiment, the nucleosides and nucleotides of the invention are fluorinated.

Advantageously, such nucleoside or nucleotide pool modulators are nucleoside or nucleotide deaminases. Deaminases are enzymes which catalyse the reaction of deamination, which is the removal of an amine group from a molecule. Nucleoside deaminases catalyse the reaction of deamination on nucleosides. Preferably, the nucleoside pool modulator for use in the invention has a cytidine deaminase activity. More preferably, it is a naturally-occurring cytidine deaminase or an analogue thereof. The term "cytidine deaminase" or "CDAse" as used herein designates cytidine deaminase and any analogue thereof, provided that such an analogue retains a substantial cytidine deaminase enzymatic activity (at least 50% of the wild-type counterpart). The term analogue encompasses fragment of CDAse (e.g. a truncated CDAse) as well as mutated CDAse comprising one or more amino acid modification(s) preferably outside the catalytic site. In the literature, cytidine deaminase is also named "deoxycytidine deaminase" and "cytidine aminohydrolase".

The cytidine deaminases belong to the family of multi-subunit enzymes that catalyze the hydrolytic deamination of their substrate to a corresponding uracil product (Somasekaram et al., 1999, J. Biol. Chem. 274 (40):28405-12). CDAses play a major role in pyrimidine nucleoside and nucleotide salvage. More precisely, CDAses catalyse the deamination of cytidine-based components to uridine-based components, like for example cytidine (C) to uridine (U), and deoxycytidine (dC) to deoxyuridine (dU) (Demontis et al., 1998, Biochim. Biophys. Acta. 1443:323-33). This deamination can be directed to other cytidine-based components. For example, the deamination of dC and 5-methyl-2'-deoxicytidine (5mdC) by CDAses results in dU and thymidine (T), which are the normal precursor for thymine triphosphate synthesis. Another example is the conversion of 5-hydroxymethyl-2'deoxycytidine (5hmdC) and 5-formy-2'deoxycytidine (5fdC) into variants of uridine, respectively 5hmdU and 5fdU, which are toxic to the cells when incorporated into DNA, as they are recognized as damaged bases (Zauri et al., 2015, Nature, 524:114-118). A further example is the deamination of chemotherapeutic nucleoside analogs such as Ara-C, 5-azacytidine and 2,2-difluorodeoxycytidine (gemcitabine), which results in a loss of their cytotoxic and antitumor activity (Fitzgerald et al., 2006, Hum. Genet., 119(3):276-83).

The two families of cytidine deaminases exhibit different biological functions, deaminating either the free cytidines, as performed by cytidine deaminase (CDA or CDD; EC 3.5.4.5), or deaminating the cytidines incorporated within the DNA or RNA polymers, as performed by the AID/APOBECs (Activation-induced deaminase, and apolipoprotein B mRNA editing catalytic polypeptide like) proteins (Mameri et al., 2016, Clin. Cancer. Res. DOI: 10.1158/1078-0432.CCR-16-0626; Conticello et al., 2005, Mol. Biol. Evol. 22:367-77).

Various cytidine deaminase can be used in the context of the present invention, including mammalian (e.g. human), insects, bacterial or lower eukaryotic cytidine deaminases. In one embodiment, the oncolytic virus of the present invention encodes or is used in combination with a CDAse of human origin. Representative examples of human cytidine deaminases include, without limitation, human CDA, also named hCD (GenBank access. NO: NM_001785; NP_001776), activation-induced cytidine deaminase (AID or AICDA, GenBank access. NO: NM_020661; EAW88615.1; BAB12721), members of the APOBEC protein family (APOBEC1 (GenBank access. NO: NM 001644; BAA23882), APOBEC2 (GenBank access. NO: NM_006789; AF161698_1), APOBEC3A (GenBank access. NO: KM_266646; AKE33285), APOBEC3B (GenBank access. NO: NM_004900; AAH53859), APOBEC3C (GenBank access. NO: NM_014508), APOBEC3D (GenBank access. NO: NM_152426), APOBEC3E (GenBank access. NO: NM_152426), APOBEC3F (GenBank access. NO: NM_145298), APOBEC3G (GenBank access. NO: NM_021822; AAH61914.1), APOBEC3H (GenBank access. NO: NM_001166003), APOBEC4 (GenBank access. NO: NM-203454.2)) and cytidine deaminase-like proteins.

Cytidine deaminase can also originate from microorganisms, like fungi, yeast, virus or bacteria. Examples of non-human cytidine deaminase originate from yeast (e.g. CDD1: GenBank access. NO: NM_001182132; EDN59461), Candida glabrata (GenBank access NO: KTB24532), Arabidopsis thaliana (Genbank access. NO: AJ005687), E. coli (GenBank access NO: NP_416648), etc.

In one embodiment, the oncolytic poxvirus as defined above encodes a CDAse polypeptide selected from
the group consisting of CDA, CDD, EC 3.5.4.5, APOBEC, AID and cytidine deaminase-like proteins.

In a preferred embodiment, the oncolytic poxvirus as defined above of the invention encodes the yeast
cytidine deaminase (CDD1), wherein said CDD1 comprises an amino acid sequence having at least 80%, preferably greater than 90%, and more preferably greater than 95% identity with SEQ ID NO:1.

In another embodiment, the oncolytic poxvirus as defined above of the invention encodes the
human cytidine deaminase (hCD), wherein said hCD comprises an amino acid sequence having at least 80%, preferably greater than 90%, and more preferably greater than 95% identity with SEQ ID NO:2.

In still another preferred embodiment, the oncolytic poxvirus as defined above of the invention encodes
the human APOBEC2, wherein said APOBEC2 comprises an amino acid sequence having at least 80%, preferably greater than 90%, and more preferably greater than 95% identity with SEQ ID NO: 3.

The present invention preferably relates to an oncolytic poxvirus as defined above comprising one or more
cytidine deaminase encoding nucleic acid molecules. For example, nucleic acid molecules encoding yeast cytidine deaminase (CDD1) and human cytidine deaminase (hCD) can be inserted in the viral genome (e.g. at different locations) under the control of appropriate regulatory elements as described hereinafter. The CDAse-encoding nucleic acid molecule(s) can be inserted at any location of the viral genome, with a specific preference for a non-essential locus. For example, TK gene, RR gene or intergenic zones are particularly appropriate for insertion in oncolytic vaccinia virus. In a preferred embodiment, the present invention relates to an attenuated vaccinia virus defective in viral TK and RR-encoded activities and encoding a human or a yeast CDAse, such as VVTK-RR-/CDD1, VVTK-RR-/hCD or VVTK-RR-/APOBEC2 illustrated in the Example section.

The "cytidine deaminase encoding nucleic acid molecule" may be easily obtained by cloning, by PCR or by chemical synthesis based on the information provided herein and the general knowledge of the skilled person. The CDAse-encoding nucleic acid molecule for use herein may be native CDAse-encoding sequences (e.g. cDNA) or analogue thereof derived from the latter by mutation, deletion, substitution and/or addition of one or more nucleotides. Moreover, the CDAse-encoding nucleic acid molecule can be optimized for providing high level expression in a particular host cell or subject as described hereinafter.

### Nucleic acid(s) of interest

According to a specific embodiment, the oncolytic poxvirus as defined above of the invention may further
comprise, inserted in its genome, one or more nucleic acid(s) of interest different from CDAse-encoding nucleic acid molecule. According to the invention, the nucleic acid(s) of interest can be homologous or heterologous to the host organism into which it is introduced. More specifically, it can be of human origin or not (e.g. of bacterial, yeast or viral origin). Advantageously, said nucleic acid of interest encodes all or part of a polypeptide. A polypeptide is understood to be any translational product of a polynucleotide regardless of size, and whether glycosylated or not, and includes peptides and proteins.

A vast number of nucleic acids of interest may be envisaged in the context of the invention such as those encoding polypeptides which are expected to cause a beneficial effect on the course or a symptom of the pathological condition to be treated, such as polypeptides that can compensate for defective or deficient proteins in the subject, or those that act through toxic effects to limit or remove harmful cells from the body or those that encode immunity conferring polypeptides (e.g. immunostimulatory polypeptides and/or antigens for inducing or activating an immune humoral and/or cellular response), or permease to increase the cellular nucleos/tide pool among many others. Such nucleic acids of interest may be obtained from a naturally-occurring sequence or modified by mutation, deletion, substitution and/or addition of one or more nucleotides.

In the context of the invention, the nucleic acid of interest can originate from Prokaryotes (comprising the kingdoms of Bacteria, Archaea), Acaryotes (comprising the viruses) or Eucaryotes (comprising the kingdoms of Protista, Fungi, Plantae, Animalia).

Examples of suitable nucleic acids of interest are given in the following list: the oncolytic poxvirus as defined above of the present invention may further carry a nucleic acid coding for
polypeptides selected in the group consisting of enzymes (urease, renin, thrombin, metalloproteinase, nitric oxide synthase NOS, SOD, catalase, hyaluronidase, ...), enzyme inhibitors (alpha1-antitrypsin, antithrombin III, viral protease inhibitor, plasminogen activator inhibitor PAI-1), proteins, MHC antigens of class I or II, polypeptides that can modulate/regulate expression of cellular genes, polypeptide capable of inhibiting a bacterial, parasitic or viral infection or its development (antigenic polypeptides, antigenic epitopes, transdominant variants inhibiting the action of a native protein by competition....), apoptosis inducers or inhibitors, (Bax, Bak, Bok, Bad, Bid et Bim, Bax inhibitor, Bak inhibitor, Bok inhibitor, Bad inhibitor, Bid inhibitor, Bim inhibitor, Bcl-2, Bcl-xL, Bcl-w, Nr13, Bcl2 inhibitor, Bcl-xL-inhibitor, Bcl-w inhibitor, Nr13 inhibitor), cytostatic agents (p21, p16, Rb...), apolipoproteins (ApoAI, ApoAIV, ApoE...), inhibitors of angiogenesis (angiostatin, endostatin...), anti-VEGF antibodies or scFv, oxygen radical scaveyers, polypeptides having an anti-tumor effect, antibodies, toxins, immunotoxins, markers (beta-galactosidase, luciferase....) or any other nucleic acids of interest that are recognized in the art as being useful for the treatment or prevention of a clinical condition. Suitable anti-tumor nucleic acids include but are not limited to those encoding tumor suppressor genes (e.g. Rb, p53, DCC, NF-1, Wilm's tumor, NM23, BRUSH-1, p56, p73 as well as their respective mutants), antibodies, polypeptides inhibiting cellular division or transduction signals.

### Immunostimulatory polypeptide

In another embodiment of the invention, the oncolytic poxvirus as defined above of the invention may
further comprise an immunostimulatory polypeptide. As used herein, the term "immunostimulatory polypeptide" refers to a polypeptide, or protein, which has the ability to stimulate the immune system, in a specific or non-specific way. A vast number of proteins are known in the art for their ability to exert an immunostimulatory effect. Examples of suitable immunostimulatory proteins in the context of the invention include, without limitation, immune checkpoint inhibitors, including, but not limited to anti-PD1, anti-PDL1, anti-PDL-2, anti-CTLA4, anti-Tim3, anti-LAG3, anti-BTLA; cytokines, like alpha, beta or gamma interferon, interleukin (in particular IL-2, IL-6, IL-10 or IL-12) or tumor necrosis factor (TNF); agents that affect the regulation of cell surface receptors such as, e.g. inhibitors of Epidermal Growth Factor Receptor (in particular cetuximab, panitumumab, zalutumumab, nimotuzumab, matuzumab, gefitinib, erlotinib or lapatinib) or inhibitors of Human Epidermal Growth Factor Receptor-2 (in particular trastuzumab); agents that affect angiogenesis such as, e.g. inhibitor of Vascular Endothelial Growth Factor (in particular bevacizumab or ranibizumab) ; agents that stimulates stem cells to produce granulocytes (neutrophils, eosinophils, and basophils), macrophages such as, e.g. granulocyte macrophage - colony stimulating factor (GM-CSF, C-CSF, M-CSF...) ), and B7 proteins (B7.1, B7.2 and the like).

### Antigens

The term "antigen" generally refers to a substance that is recognized and selectively bound by an antibody or by a T cell antigen receptor, in order to trigger an immune response. It is contemplated that the term antigen encompasses native antigen as well as fragment (e.g. epitopes, immunogenic domains, etc.) and derivative thereof, provided that such fragment or derivative is capable of being the target of an immune response. Suitable antigens in the context of the invention are preferably polypeptides (e.g. peptides, polypeptides, post translationally modified polypeptides, etc.) including one or more B cell epitope(s) or one or more T cell epitope(s) or both B and T cell epitope(s) and capable of raising an immune response, preferably, a humoral or cell response that can be specific for that antigen. Typically, the one or more antigen(s) is selected in connection with the disease to treat. Preferred antigens for use herein are cancer antigens and antigens of tumor-inducing pathogens.

In certain embodiments, the antigen(s) contained in or encoded by the oncolytic virus is/are cancer antigen(s) (also called tumor-associated antigens) that is associated with and/or serve as markers for cancers. Cancer antigens encompass various categories of polypeptides, e.g. those which are normally silent (i.e. not expressed) in normal cells, those that are expressed only at low levels or at certain stages of differentiation and those that are temporally expressed such as embryonic and foetal antigens as well as those resulting from mutation of cellular genes, such as oncogenes (e.g. activated ras oncogene), proto-oncogenes (e.g. ErbB family), or proteins resulting from chromosomal translocations. The cancer antigens also encompass antigens encoded by pathogenic organisms (bacteria, viruses, parasites, fungi, viroids or prions) that are capable of inducing a malignant condition in a subject (especially chronically infected subject) such as RNA and DNA tumor viruses (e.g. HPV, HCV, EBV, etc.) and bacteria (e.g. *Helicobacter pilori*).

Some non-limiting examples of cancer antigens include, without limitation, MART-1/Melan-A, gp100, Dipeptidyl peptidase IV (DPPIV), adenosine deaminase-binding protein (ADAbp), cyclophilin b, Colorectal associated antigen (CRC)-C017-1A/GA733, Carcinoembryonic Antigen (CEA) and its immunogenic epitopes CAP-1 and CAP-2, etv6, aml1, Prostate Specific Antigen (PSA) and its immunogenic epitopes PSA-1, PSA-2, and PSA-3, prostate-specific membrane antigen (PSMA), T-cell receptor/CD3-zeta chain, MAGE-family of tumor antigens (e.g., MAGE-A1, MAGE-A2, MAGE-A3, MAGE-A4, MAGE-A5, MAGE-A6, MAGE-A7, MAGE-A8, MAGE-A9, MAGE-A10, MAGE-A11, MAGE-A12, MAGE-Xp2 (MAGE-B2), MAGE-Xp3 (MAGE-B3), MAGE-Xp4 (MAGE-B4), MAGE-C1, MAGE-C2, MAGE-C3, MAGE-C4, MAGE-C5), GAGE-family of tumor antigens (e.g., GAGE-1, GAGE-2, GAGE-3, GAGE-4, GAGE-5, GAGE-6, GAGE-7, GAGE-8, GAGE-9), BAGE, RAGE, LAGE-1, NAG, GnT-V, MUM-1, CDK4, tyrosinase, p53, MUC family (e.g. MUC1, MUC16, etc.; see e.g. US6,054,438; WO98/04727; or WO98/37095), HER2/neu, p21ras, RCAS1, alpha-fetoprotein, E-cadherin, alpha-catenin, beta-catenin and gamma-catenin, p120ctn, gp100.sup.Pmel117, PRAME, NY-ESO-1, cdc27, adenomatous polyposis coli protein (APC), fodrin, Connexin 37, Ig-idiotype, p15, gp75, GM2 and GD2 gangliosides, Smad family of cancer antigens brain glycogen phosphorylase, SSX-1, SSX-2 (HOM-MEL-40), SSX-1, SSX-4, SSX-5, SCP-1 and CT-7, and c-erbB-2 and viral antigens such as the HPV-16 and HPV-18 E6 and E7 antigens and the EBV-encoded nuclear antigen (EBNA)-1.

Other antigens suitable for use in this invention are marker antigens (beta-galactosidase, luciferase, green fluorescent proteins, etc.).

The present invention also encompasses oncolytic poxviruses as defined above expressing two or more
polypeptides of interest as described herein, e.g. at least two antigens, at least one antigen and one cytokine, at least two antigens and one cytokine, etc.

### Permeases

According to another embodiment, the oncolytic poxvirus as defined above of the invention may further
comprise at least one nucleic acid of interest which is a permease.

As used herein, the term "permease" refers to trans-membranous protein involved in the translocation of nucleoside and nucleobases. Examples of permeases which are involved in the translocation of nucleosides, nucleoside analogues and nucleobases are hCNT1, hCNT2, hCNT3, hENT1 and hENT2. hCNT1, hCNT2 and hCNT3 proteins translocate nucleosides in a Na+ coupled manner with high affinity and some substrate selectivity, being hCNT1 and hCNT2 pyrimidine - and purine - preferring, respectively, and hCNT3 abroad selectivity transporter. hENT1 and hENT2 are unequivocally implicated in the translocation of nucleosides and nucleobases (Pastor-Anglada et al, 2015, Front. Pharmacol., 6(13):1-14).

Other nucleic acids of interest:
The oncolytic poxvirus of the invention may further comprise at least one nucleic acid of interest including, but not limited to:
   - nucleic acid coding for endonuclease, like restriction enzymes (e.g. restriction enzymes of type I, II, III, IV or V, artificial restriction enzymes like Transcription activator-like effector nucleases (TALEN) or zinc finger nuclease), CRISPR/Cas9;
   - RNA, including but not limited to target-specific miRNA, shRNA, siRNA.
The nucleic acids of interest sequences may be easily obtained by cloning, by PCR or by chemical synthesis using conventional techniques. Moreover, their sequences are widely described in the literature which can be consulted by persons skilled in the art. As for the CDAse-encoding nucleic acid molecule, the nucleic acid(s) of interest can be inserted at any location of the viral genome, with a specific preference for a non-essential locus. For example, TK gene, RR gene or intergenic zones are particularly appropriated for insertion in oncolytic vaccinia virus.

### Expression of the CDAse-encoding nucleic acid molecule and of the nucleic acid(s) of interest

As mentioned before the CDAse-encoding nucleic acid molecule(s) and/or the nucleic acid(s) of interest to be inserted in the genome of the oncolytic poxvirus of the present invention can be optimized for providing high level expression in a particular host cell or subject. It has been indeed observed that, the codon usage patterns of organisms are highly non-random and the use of codons may be markedly different between different hosts. As such nucleic acid(s) might be from bacterial or lower eukaryote origin, they may have an inappropriate codon usage pattern for efficient expression in higher eukaryotic cells (e.g. human). Typically, codon optimization is performed by replacing one or more "native" (e.g. bacterial or yeast) codon corresponding to a codon infrequently used in the host organism of interest by one or more codon encoding the same amino acid which is more frequently used. It is not necessary to replace all native codons corresponding to infrequently used codons since increased expression can be achieved even with partial replacement.

Further to optimization of the codon usage, expression in the host cell or subject can further be improved through additional modifications of the nucleic acid sequence. For example, it may be advantageous to prevent clustering of rare, non-optimal codons being present in concentrated areas and/or to suppress or modify "negative" sequence elements which are expected to negatively influence expression levels. Such negative sequence elements include without limitation the regions having very high (>80%) or very low (<30%) GC content; AT -rich or GC-rich sequence stretches; unstable direct or inverted repeat sequences; and/or internal cryptic regulatory elements such as internal TATA-boxes, chi-sites, ribosome entry sites, and/or splicing donor/acceptor sites.

In accordance with the present invention, the oncolytic poxvirus as defined above comprises the elements
necessary for the expression of the nucleic acid(s) of interest. More precisely, the CDAse-encoding nucleic acid molecule(s) and/or the nucleic acid(s) of interest inserted in the genome of the oncolytic virus of the invention is/are operably linked to suitable regulatory elements for its/their expression in a host cell or subject. As used herein, the term "regulatory elements" or "regulatory sequence" refers to any element that allows, contributes or modulates expression in a given host cell or subject, including replication, duplication, transcription, splicing, translation, stability and/or transport of the nucleic acid(s) or its derivative (i.e. mRNA). As used herein, "operably linked" means that the elements being linked are arranged so that they function in concert for their intended purposes. For example, a promoter is operably linked to a nucleic acid molecule if the promoter effects transcription from the transcription initiation to the terminator of said nucleic acid molecule in a permissive host cell.

It will be appreciated by those skilled in the art that the choice of the regulatory sequences can depend on such factors as the nucleic acid molecule itself, the virus into which it is inserted, the host cell or subject, the level of expression desired, etc. The promoter is of special importance. In the context of the invention, it can be constitutive directing expression of the encoded product (e.g. CDAse and/or therapeutic molecule) in many types of host cells or specific to certain host cells (e.g. liver-specific regulatory sequences) or regulated in response to specific events or exogenous factors (e.g. by temperature, nutrient additive, hormone, etc.) or according to the phase of a viral cycle (e.g. late or early). One may also use promoters that are repressed during the production step in response to specific events or exogenous factors, in order to optimize virus production and circumvent potential toxicity of the expressed polypeptide(s).

Although conventional promoters such as cytomegalovirus (CMV) immediate early promoter (US 5,168,062), the RSV promoter, the adenovirus major late promoter, the phosphoglycero kinase (PGK) promoter (Adra et al., 1987, Gene 60: 65-74), the thymidine kinase (TK) promoter of herpes simplex virus (HSV)-1 and the T7 polymerase promoter (WO98/10088) may be used in the context of the present invention. Vaccinia virus promoters are particularly adapted for expression in oncolytic poxviruses. Representative examples include without limitation the vaccinia 7.5K, H5R, 11K7.5 (Erbs et al., 2008, Cancer Gene Ther. 15(1): 18-28), TK, p28, p11 Pr13.5 (WO2014/063832), pB8R, pF11L, pA44L, pC11R (WO2011/128704) and K1L promoter, as well as synthetic promoters such as those described in Chakrabarti et al. (1997, Biotechniques, 23: 1094-7; Hammond et al., 1997, J. Virol. Methods, 66: 135-8; and Kumar and Boyle, 1990, Virology, 179: 151-8) as well as early/late chimeric promoters (e.g. US 8,394,385; US 8,772,023). Cowpox promoters are also suitable as well (e.g. the ATI promoter). In a preferred embodiment, the CDAse nucleic acid molecule is inserted in the TK locus of the oncolytic virus of the invention and placed under the control of the vaccinia p11.5K promoter.

Those skilled in the art will appreciate that the regulatory elements controlling the nucleic acid expression may further comprise additional elements for proper initiation, regulation and/or termination of transcription (e.g. a transcription termination sequences), mRNA transport (e.g. nuclear localization signal sequences), processing (e.g. splicing signals), and stability (e.g. introns and non-coding 5' and 3' sequences), translation (e.g. an initiator Met, tripartite leader sequences, IRES ribosome binding sites, signal peptides, etc.), targeting sequences, transport sequences, secretion signal, and sequences involved in replication or integration. Said sequences have been reported in the literature and can be readily obtained by those skilled in the art.

### Process for preparing an oncolytic virus

The invention also relates to a process for preparing an oncolytic poxvirus as defined above of the invention, in which process:
(i) an oncolytic poxvirus of the invention is introduced into a producer cell,
(ii) said producer cell is cultured under conditions which are appropriate for enabling said oncolytic poxvirus to be produced, and
(iii) said oncolytic poxvirus is recoverd from the cell culture.

Typically, the oncolytic poxvirus of the present invention is produced into a suitable host cell line using conventional techniques including culturing the transfected or infected host cell under suitable conditions so-as to allow the production of infectious viral particles and recovering the produced infectious viral particles from the culture of said cell and optionally purifying said recovered infectious viral particles. Suitable host cells for production of the oncolytic virus include without limitation human cell lines such as HeLa (ATCC), 293 cells (Graham et al., 1997, J. Gen. Virol. 36: 59-72), HER96, PER-C6 (Fallaux et al., 1998, Human Gene Ther. 9: 1909-17), Monkey cells such as Vero (ATCC CCL-081), CV1(ATCC CCL-70) and BSC1(ATCC CCL-26) cell lines, avian cells such as those described in WO2005/042728, WO2006/108846, WO2008/129058, WO2010/130756, WO2012/001075, etc.), hamster cell lines such as BHK-21 (ATCC CCL-10) as well as primary chicken embryo fibroblasts (CEF) prepared from chicken embryos obtained from fertilized eggs. Host cells are preferably cultivated in a medium free of animal-or human-derived products, using a chemically defined medium with no product of animal or human origin. Culturing is carried out at a temperature, pH and oxygen content appropriate for the producer cell. Such culturing conditions are within the expertise of one of ordinary skill in the art. If growth factors are present, they are preferably recombinantly produced and not purified from animal material. Suitable animal-free medium media are commercially available, for example VP-SFM medium (Invitrogen) for culturing CEF producer cells. Producer cells are preferably cultivated at a temperature comprised between +30°C and +38°C (more preferably at about +37°C) for between 1 and 8 days (preferably for 1 to 5 days for CEF and 2 to 7 days for immortalized cells) before infection. If needed, several passages of 1 to 8 days may be made in order to increase the total number of cells.

Producer cells are infected by the oncolytic virus with an appropriate multiplicity of infection (MOI), which can be as low as 0.001 (more preferably between 0.05 and 5) to permit productive infection.

In step ii), infected producer cells are then cultured under appropriate conditions well known to those skilled in the art until progeny viral vector is produced. Culture of infected producer cells is also preferably performed in a chemically defined medium (which may be the same as or different from the medium used for culture of producer cells and/or for infection step) free of animal- or human-derived products at a temperature between +30°C and +37°C, for 1 to 5 days.

In step iii), the viral particles may be collected from the culture supernatant and/or the producer cells. Recovery from producer cells (and optionally also from culture supernatant), may require a step allowing the disruption of the producer cell membrane to allow the liberation of the virus from producer cells. The disruption of the producer cell membrane can be induced by various techniques well known to those skilled in the art, including but not limited to, freeze/thaw, hypotonic lysis, sonication, microfluidization, or high-speed homogenization.

The recovered oncolytic poxvirus as defined above can be at least partially purified before being used
according to the present invention. Various purification steps can be envisaged, including clarification, enzymatic treatment (e.g. endonuclease such as benzonase, protease), ultracentrifugation (e.g. sucrose gradient or cesium chloride gradient), chromatographic and filtration steps. Appropriate methods are described in the art (e.g. WO2007/147528; WO2008/138533, WO2009/100521, WO2010/130753, WO2013/022764).

### Oncolytic virus composition

The invention also relates to a composition which comprises a therapeutically effective amount of the oncolytic poxvirus as defined above of the present invention or prepared according to
the process described herein. Preferably, the composition further comprises a pharmaceutically acceptable vehicle.

A "therapeutically effective amount" corresponds to the amount of each of the active agents comprised in the composition of the invention that is sufficient for producing one or more beneficial results. Such a therapeutically effective amount may vary as a function of various parameters, e.g. the mode of administration; the disease state; the age and weight of the subject; the ability of the subject to respond to the treatment; kind of concurrent treatment; the frequency of treatment; and/or the need for prevention or therapy. When prophylactic use is concerned, the composition of the invention is administered at a dose sufficient to prevent or to delay the onset and/or establishment and/or relapse of a pathologic condition (e.g. a proliferative disease such as cancer), especially in a subject at risk. For "therapeutic" use, the composition of the invention is administered to a subject diagnosed as having a pathological condition (e.g. a proliferative disease such as cancer) with the goal of treating the disease, optionally in association with one or more conventional therapeutic modalities. In particular, a therapeutically effective amount could be that amount necessary to cause an observable improvement of the clinical status over the baseline status or over the expected status if not treated, as described hereinafter. An improvement of the clinical status can be easily assessed by any relevant clinical measurement typically used by physicians and skilled healthcare staff. For example, techniques routinely used in laboratories (e.g. flow cytometry, histology, imaging techniques, etc.) may be used to perform tumor surveillance. A therapeutically effective amount could also be the amount necessary to cause the development of an effective non-specific (innate) and/or specific anti-tumor response. Typically, development of an immune response in particular T cell response can be evaluated in vitro, in suitable animal models or using biological samples collected from the subject. One may also use various available antibodies so-as to identify different immune cell populations involved in anti-tumor response that are present in the treated subjects, such as cytotoxic T cells, activated cytotoxic T cells, natural killer cells and activated natural killer cells.

The appropriate dosage of oncolytic virus can be adapted as a function of various parameters and may be routinely determined by a practitioner in the light of the relevant circumstances. Suitably, individual doses for the oncolytic poxvirus as defined above may vary within a range
extending from approximately 10³ to approximately 10¹² vp (viral particles), iu (infectious unit) or PFU (plaque-forming units) depending on the virus and the quantitative technique used. For illustrative purposes, a suitable dose of oncolytic vaccinia virus for human use is comprised between approximately 10⁴ to approximately 10¹¹ PFU, preferably between approximately 10⁵ PFU to approximately 10¹⁰ PFU; doses of approximately 10⁶ PFU to approximately 5×10⁹ PFU being particularly preferred (e.g. dose of 10⁶, 2×10⁶, 3×10⁶, 4×10⁶, 5×10⁶, 6×10⁶, 7×10⁶, 8×10⁶, 9×10⁶, 10⁷, 2×10⁷, 3×10⁷, 4×10⁷, 5×10⁷, 6×10⁷, 7×10⁷, 8×10⁷, 9×10⁷, 10⁸, 2×10⁸, 3×10⁸, 4×10⁸, 5×10⁸, 6×10⁸, 7×10⁸, 8×10⁸, 9×10⁸, 10⁹, 2×10⁹, 3×10⁹, 4×10⁹ or 5×10⁹ PFU). The quantity of virus present in a sample can be determined by routine titration techniques, e.g. by counting the number of plaques following infection of permissive cells (e.g. BHK-21 or CEF), immunostaining (e.g. using anti-virus antibodies; Caroll et al., 1997, Virology 238: 198-211), by measuring the A260 absorbance (vp titers), or still by quantitative immunofluorescence (iu titers).

The term "pharmaceutically acceptable vehicle" is intended to include any and all carriers, solvents, diluents, excipients, adjuvants, dispersion media, coatings, antibacterial and antifungal agents, absorption agents and the like compatible with administration in mammals and in particular human subjects.

The oncolytic poxvirus of the invention can independently be placed in a solvent or diluent appropriate for human or animal use. The solvent or diluent is preferably isotonic, hypotonic or weakly hypertonic and has a relatively low ionic strength. Representative examples include sterile water, physiological saline (e.g. sodium chloride), Ringer's solution, glucose, trehalose or saccharose solutions, Hank's solution, and other aqueous physiologically balanced salt solutions (see for example the most current edition of Remington: The Science and Practice of Pharmacy, A. Gennaro, Lippincott, Williams&Wilkins).

In other embodiments, oncolytic poxviruses are suitably buffered for human use. Suitable buffers include without limitation phosphate buffer (e.g. PBS), bicarbonate buffer and/or Tris buffer capable of maintaining a physiological or slightly basic pH (e.g. from approximately pH 7 to approximately pH 9).

The composition of the invention may also contain other pharmaceutically acceptable excipients for providing desirable pharmaceutical or pharmacodynamic properties, including for example osmolarity, viscosity, clarity, colour, sterility, stability, rate of dissolution of the formulation, modifying or maintaining release or absorption into a human or animal subject, promoting transport across the blood barrier or penetration in a particular organ.

In a further embodiment, the composition of the invention may be adjuvanted to further enhance immunity (especially a T cell-mediated immunity) or facilitate infection of tumor cells upon administration. Representative examples of suitable adjuvants include, without limitation, alum, mineral oil emulsion such as, Freunds complete and incomplete (IFA), lipopolysaccharide or a derivative thereof (Ribi et al., 1986, Plenum Publ. Corp.,407-419), saponins such as QS21 (Sumino et al., 1998, J.Virol. 72: 4931; WO98/56415), imidazo-quinoline compounds such as Imiquimod (Suader, 2000, J. Am Acad Dermatol. 43:S6), S-27609 (Smorlesi, 2005, Gene Ther. 12: 1324) and related compounds such as those described in WO2007/147529, polysaccharides such as Adjuvax and squalenes, oil in water emulsions such as MF59, double-stranded RNA analogs such as poly(I:C), single stranded cytosine phosphate guanosine oligodeoxynucleotides (CpG) (Chu et al., 1997, J. Exp. Med., 186: 1623; Tritel et al., 2003, J. Immunol., 171: 2358) and cationic peptides such as IC-31 (Kritsch et al., 2005, J. Chromatogr. Anal. Technol. Biomed. Life Sci., 822: 263-70).

In one embodiment, the composition of the invention may be formulated with the goal of improving its stability, in particular under the conditions of manufacture and long-term storage (i.e. for at least 6 months, with a preference for at least two years) at freezing (e.g. -70°C, -20°C), refrigerated (e.g. 4°C) or ambient temperatures. Various virus formulations are available in the art either in frozen, liquid form or lyophilized form (e.g. WO98/02522, WO01/66137, WO03/053463, WO2007/056847 and WO2008/114021, etc.). Solid (e.g. dry powdered or lyophilized) compositions can be obtained by a process involving vacuum drying and freeze-drying. For illustrative purposes, buffered formulations including NaCl and/or sugar are particularly adapted to the preservation of viruses (e.g. Tris 10 mM pH 8 with saccharose 5 % (W/V), Sodium glutamate 10 mM, and NaCl 50 mM or phosphate-buffered saline with glycerol (10%) and NaCl).

The oncolytic poxvirus composition is preferably formulated in a way adapted to the mode of administration to ensure proper distribution and release in vivo. For example, gastro-resistant capsules and granules are particularly appropriate for oral administration, suppositories for rectal or vaginal administration, optionally in combination with absorption enhancers useful to increase the pore size of the mucosal membranes. Such absorption enhancers are typically substances having structural similarities to the phospholipid domains of the mucosal membranes (such as sodium deoxycholate, sodium glycocholate, dimethyl-beta-cyclodextrin, lauryl-1-lysophosphatidylcholine). Another and particularly appropriate example is a formulation adapted to the administration through microneedle means (e.g. transcutaneous or intradermal patches). Such a formulation may comprise resuspension of the immunotherapeutic product in endotoxin-free phosphate-buffered saline (PBS).

### Administration

The oncolytic poxvirus as defined above, or the composition of the invention, may be administered in a single dose or multiple doses. If multiple doses are contemplated, administrations may be
performed by the same or different routes and may take place at the same site or at alternative sites. Intervals between each administration can be from several hours to 8 weeks (e.g. 24h, 48h, 72h, weekly, every two or three weeks, monthly, etc.). Intervals can also be irregular. It is also possible to proceed via sequential cycles of administrations that are repeated after a rest period (e.g. cycles of 3 to 6 weekly administrations followed by a rest period of 3 to 6 weeks). The dose can vary for each administration within the range described above.

Any of the conventional administration routes are applicable in the context of the invention including parenteral, topical or mucosal routes. Parenteral routes are intended for administration as an injection or infusion and encompass systemic as well as local routes. Common parenteral injection types are intravenous (into a vein), intra-arterial (into an artery), intradermal (into the dermis), subcutaneous (under the skin), intramuscular (into muscle) and intratumoral (into a tumor or at its proximity). Infusions typically are given by intravenous route. Topical administration can be performed using transdermal means (e.g. patch and the like). Mucosal administrations include without limitation oral/alimentary, intranasal, intratracheal, intrapulmonary, intravaginal or intra-rectal route. In the case of intranasal, intrapulmonary and intratracheal routes, it is advantageous for administration to take place by means of an aerosol or by means of instillation. Preferred routes of administration for the oncolytic poxvirus of the invention include intravenous and intratumoral routes.

Administrations may use conventional syringes and needles (e.g. Quadrafuse injection needles) or any compound or device available in the art capable of facilitating or improving delivery of the active agent(s) in the subject. Transdermal systems are also appropriate, e.g. using solid, hollow, coated or dissolvable microneedles (see e.g., Van der Maaden et al., 2012, J. Control release 161: 645-55) and preferred are silicon and sucrose microneedle patches (see, e.g., Carrey et al., 2014, Sci Rep 4: 6154 doi 10.1038; and Carrey et al., 2011, PLoS ONE, 6(7) e22442).

A particularly preferred composition comprises 10⁶ PFU to 5×10⁹ PFU of an oncolytic poxvirus comprising a CDAse-encoding nucleic acid molecule and preferably an oncolytic
vaccinia virus defective in J2R locus (TK-), in I4L and/or F4L locus (RR-) or both in J2R locus (TK-) and in I4L/ F4L locus (TK- RR-), with a specific preference for a vaccinia having the CDAse nucleic acid molecule inserted in place of the TK locus and placed under the p11.5K promoter such as VVTK-RR-/CDD1 or VVTK-RR-/hCD described herein; formulated for intravenous or intratumoral administration.

### Methods and use

In another aspect, the present invention provides an oncolytic poxvirus or a composition thereof for use for treating or preventing a disease or a pathologic condition in a subject in need thereof. There is also disclosed a method of treatment comprising administering the oncolytic virus or the composition thereof in an amount sufficient for treating or preventing a disease or a pathologic condition in a subject in need thereof.

A "disease" (and any form of disease such as "disorder" or "pathological condition") is typically characterized by identifiable symptoms.

Examples of diseases that may be prevented or treated using the oncolytic poxvirus of the invention or the composition thereof include proliferative diseases such as cancers, tumors or restenosis and diseases associated to an increased osteoclast activity such as rheumatoid arthritis and osteoporosis.

The present invention is particularly suited for treating or preventing cancers, and particularly Adrenocortical Carcinoma, Adrenal Cortex Cancer, Anal Cancer, Gastrointestinal Carcinoid Tumors (for example Appendix Cancer and Carcinoid Tumor), Bile Duct Cancer (for example Cholangiocarcinoma), Bladder Cancer, Bone Cancer (for example Ewing Sarcoma, Malignant Fibrous Histiocytoma of Bone and Osteosarcoma), Brain Tumors (for example Astrocytomas, Embryonal Tumors, Germ Cell Tumors, Central Nervous System Atypical Teratoid/Rhabdoid Tumor, Craniopharyngioma, Ependymoma, Gliomas and Glioblastoma), Breast Cancer (for example Ductal Carcinoma In Situ), Bronchial Tumors, Carcinoma of Unknown Primary, Cardiac (Heart) Tumors, Cervical Cancer, Chordoma, Chronic Myeloproliferative Neoplasms, Colorectal Cancer (for example Rectal Cancer), Esthesioneuroblastoma, Extracranial Germ Cell Tumor, Extragonadal Germ Cell Tumor, Retinoblastoma, Gallbladder Cancer, Gastrointestinal Carcinoid Tumor, Testicular Cancer, Gestational Trophoblastic Disease, Head and Neck Cancer (for example Hypopharyngeal Cancer, pharyngeal Cancer, Laryngeal Cancer, Lip and Oral Cavity Cancer, Metastatic Squamous Neck Cancer with Occult Primary, Mouth Cancer, Nasal Cavity and Paranasal Sinus Cancer, Nasopharyngeal Cancer, Salivary Gland Cancer, Throat Cancer, Esophageal Cancer), Hepatocellular (Liver) Cancer, Histiocytosis, Langerhans Cell, Kidney cancer (for example Wilms Tumor, Renal Cell Cancer, Transitional Cell Cancer of the Renal Pelvis and Ureter), Langerhans Cell Histiocytosis, Laryngeal Cancer and Papillomatosis, Leukemia (for example Hairy Cell Leukemia, Chronic Lymphocytic Leukemia (CLL), Chronic Myelogenous Leukemia (CML), Acute Myeloid Leukemia (AML), Acute Lymphoblastic Leukemia (ALL)), Liver Cancer, Lung Cancer (Small Cell Lung Cancer and Non-Small Cell Lung Cancer), Lymphoma (for example AIDS-Related Lymphoma, Primary CNS Lymphoma, Cutaneous T-Cell Lymphoma, Hodgkin Lymphoma, Burkitt Lymphoma, Primary Lymphoma, Mycosis Fungoides, Non-Hodgkin Lymphoma, Macroglobulinemia, Waldenström, Primary Central Nervous System (CNS) Lymphoma, Sézary Syndrome, T-Cell Lymphoma), Intraocular Melanoma, Mesothelioma, Midline Tract Carcinoma Involving NUT Gene, Multiple Endocrine Neoplasia Syndromes, Multiple Myeloma/Plasma Cell Neoplasms Myelodysplastic Syndromes, Chronic Myeloproliferative Neoplasms, Neuroblastoma, Ovarian Cancer (for example Primary Peritoneal Cancer and Fallopian Tube Cancer), Pancreatic Cancer and Pancreatic Neuroendocrine Tumors (Islet Cell Tumors), Papillomatosis, Paraganglioma, Parathyroid Cancer, Penile Cancer, Pheochromocytoma, Pituitary Tumor, Plasma Cell Neoplasm/Multiple Myeloma, Pleuropulmonary Blastoma, Prostate Cancer, Retinoblastoma, Vascular Tumors, Skin Cancer (for example Basal Cell Carcinoma, Melanoma, Squamous Cell Carcinoma and Merkel Cell Carcinoma), Small Intestine Cancer, Soft Tissue Sarcoma (for example Gastrointestinal Stromal Tumors (GIST), AIDS-Related Cancers Kaposi Sarcoma, Kaposi Sarcoma and Rhabdomyosarcoma), Stomach (Gastric) Cancer, Testicular Cancer, Thymoma and Thymic Carcinoma, Thyroid Cancer, Urethral Cancer, Uterine Cancer, Endometrial and Uterine Sarcoma, Vaginal Cancer and Vulvar Cancer. The present invention is also useful for treatment of metastatic cancers.

In a preferred embodiment, the oncolytic poxvirus or a composition of the invention is used for treating gastro-intestinal cancers, comprising cancers of oesophagus, gallbladder, liver, pancreas, stomach, small intestine, bowel (large intestine or colon and rectum), and anus. A particularly preferred method comprises 1 to 6 intravenous or intratumoral administrations of the oncolytic poxvirus of the invention or composition thereof given at weekly to monthly intervals with a specific preference for 3 bi-weekly administrations (e.g. at approximately D1, D14 and D29) of a composition comprising 10⁶-5×10⁹ PFU of an oncolytic vaccinia virus comprising inserted in its genome a CDAse-encoding nucleic acid molecule (e.g. placed under the p11.5K promoter), preferably defective in J2R locus (TK-), in I4L and/or F4L locus (RR-) or both in J2R locus (TK-) and in I4L/ F4L locus (TK- RR-), such as VVTK-RR-/CDD1 or VVTK-RR-/hCD.

The beneficial effects provided by the methods of the present invention can be evidenced by an observable improvement of the clinical status over the baseline status or over the expected status if not treated according to the modalities described herein. An improvement of the clinical status can be easily assessed by any relevant clinical measurement typically used by physicians and skilled healthcare staff. In the context of the invention, the therapeutic benefit can be transient (for one or a couple of months after cessation of administration) or sustained (for several months or years). As the natural course of clinical status which may vary considerably from a subject to another, it is not required that the therapeutic benefit be observed in each subject treated but in a significant number of subjects (e.g. statistically significant differences between two groups can be determined by any statistical test known in the art, such as a Tukey parametric test, the Kruskal-Wallis test the U test according to Mann and Whitney, the Student's t-test, the Wilcoxon test, etc.).

In a particular embodiment, as the methods according to the present invention are particularly appropriate for treating cancer, such methods can be correlated with one or more of the followings: inhibiting or slowing tumor growth, proliferation and metastasis, preventing or delaying tumor invasion (spread of tumor cells in neighbouring tissues), reducing the tumor number; reducing the tumor size, reducing the number or extent of metastases, providing a prolonged overall survival rate (OS), increasing progression free survival (PFS), increasing the length of remission, stabilizing (i.e. not worsening) the state of disease, providing a better response to the standard treatment, improving quality of life and/or inducing an anti-tumor response (e.g. non-specific (innate) and/or specific such as a cytotoxic T cell response) in the subject treated in accordance with the present invention.

The appropriate measurements that can be used to assess a clinical benefit such as blood tests, analysis of biological fluids and biopsies as well as medical imaging techniques are evaluated routinely in medical laboratories and hospitals and a large number of kits is available commercially. They can be performed before the administration (baseline) and at various time points during treatment and after cessation of the treatment.

There is also disclosed a method for treating a disease or a pathologic condition in a subject in need thereof comprising administering the oncolytic poxvirus or the composition of the present invention, or prepared according to the process described herein. Said disease may be a proliferative disease such as cancers, tumors and restenosis. Said disease may be a disease associated with an increased osteoclast activity like rheumatoid arthritis and osteoporosis. More precisely,
there is disclosed a method for inhibiting tumor cell growth in vivo comprising administering an oncolytic virus or a composition thereof in a subject in need thereof so-as to inhibit the growth of a tumor. For general guidance, inhibition of tumor cell growth can be evaluated routinely, for example by radiography means. The administration(s) of the oncolytic virus or a composition thereof desirably result(s) in at least a 10% decrease of the tumor mass.

### Combination therapy

The oncolytic poxvirus or composition can be administered in association with any conventional therapeutic modalities which are available for treating or preventing the targeted disease or pathological condition. Representative examples of conventional therapies include, without limitation, surgery, radiotherapy, chemotherapy, cryotherapy, hormonal therapy, toxin therapy, immunotherapy, cytokine therapy, transplantation (e.g. stem cell), hyperthermia and photodynamic therapy. Such conventional therapy/ies is/are administered to the subject in accordance with standard practice before, after, essentially simultaneously or in an interspersed manner with the oncolytic virus or composition thereof.

In one embodiment, the oncolytic poxvirus or composition according to the invention can be used in association with radiotherapy. Those skilled in the art can readily formulate appropriate radiation therapy protocols and parameters (see for example Perez and Brady, 1992, Principles and Practice of Radiation Oncology, 2nd Ed. JB Lippincott Co; using appropriate adaptations and modifications as will be readily apparent to those skilled in the field). The types of radiation that may be used are well known in the art and include electron beams, high-energy photons from a linear accelerator or from radioactive sources such as cobalt or cesium, protons, and neutrons. Dosage ranges for radio-isotopes can be defined by the practitioners depending on the half-life of the isotope, the strength and type of radiation emitted, and the uptake by the neoplastic cells. Regular X-rays doses for prolonged periods of time (3 to 6 weeks), or high single doses are contemplated by the present invention.

The poxvirus or composition as defined above may be used in conjunction with surgery. For example, the oncolytic poxvirus or the composition thereof may be administerd upon excision of
the tumor (e.g. by local application within the excised zone for example).

The oncolytic poxvirus or composition thereof may be used in combination with one or more substances effective in anticancer therapy, like chemotherapeutic drugs or immunotherapeutic products.

In a specific embodiment, the oncolytic poxvirus or composition of the invention may be used in conjunction with chemotherapeutic drugs currently used for treating cancer. Although any anti-cancer chemotherapy drug may be used in combination with the oncolytic poxvirus of the present invention or the composition thereof, there may be mentioned more specifically alkylating agents, topoisomerase I inhibitors, topoisomerase II inhibitors, platinum derivatives, inhibitors of tyrosine kinase receptors, antimetabolites and antimitotic agents.

In still further embodiments, the oncolytic poxvirus or composition of the invention may be used in conjunction with immunotherapy, and especially with anti-neoplastic antibodies as well as siRNA and antisense polynucleotides. Representative examples include, among others, monoclonal antibodies blocking immune checkpoint (e.g. Ipilimumab, tremelimumab pembrolizumab, nivolumab, pidilizumab, AMP-224MEDI4736, MPDL3280A, BMS-936559, etc.), alpha, beta or gamma interferon, interleukin (in particular IL-2, IL-6, IL-10 or IL-12) or tumor necrosis factor; agents that affect the regulation of cell surface receptors such as, e.g. monoclonal antibodies blocking Epidermal Growth Factor Receptor (in particular cetuximab, panitumumab, zalutumumab, nimotuzumab, matuzumab, trastuzumab (Herceptin™), gefitinib, erlotinib, lapatinib etc.) and monoclonal antibodies blocking Vascular Endothelial Growth Factor (in particular bevacizumab and ranibizumab). Other representative examples of such immunotherapeutic products suitable for use in the present invention are plasmid DNA vector, vaccinia virus (e.g. Copenhagen, WR, Wyeth, MVA, etc.), adenovirus, lentivirus, herpes virus, recombinant polypeptides, among many others.

Another aspect as disclosed herein concerns the combination of an oncolytic poxvirus (e.g. wild type oncolytic poxvirus, or modified derivative oncolytic virus) with at least one cytidine deaminase, wherein said cytidine deaminase is not encoded by the oncolytic poxvirus. Cytidine
deaminase, wherein said cytidine deaminase is not encoded by the oncolytic virus. Cytidine deaminase may be encoded by another vector, like for example DNA molecules (plasmids, non-oncolytic viral vectors, cosmids and artificial chromosomes), RNA molecules, nanoparticles, etc. Cytidine deaminase may also be in its polypeptide form. The polypeptide can be of any origin, e.g. human, humanized, animal, insects, microorganisms or chimeric. In addition, the polypeptide may be glycosylated or non-glycosylated.

The oncolytic poxvirus or composition thereof may also be used in combination with substances which enhance cytidine deaminase activity (e.g. enhancers of the expression of cytidine deaminase, inhibitors of DNA methylation, like 5-Aza-dZ).

One may provide the subject with the oncolytic poxvirus and the additional anti-cancer therapy sequentially or in an interspersed way but concomitant administrations of both therapies within the same period of time are also contemplated. The course of treatment may be routinely determined by a practitioner and various protocols are encompassed by the present invention. For example, 1 to 10 administrations of the oncolytic poxvirus (e.g. 3 to
6 biweekly injections) may be interspersed within one or multiple administrations of the additional anti-cancer therapy (for example chemotherapy may be given in one or more cycles of one or several weeks). Moreover, after a course of treatment, it is contemplated that there is a period of time at which no anti-cancer treatment is administered before repetition of treatment cycle(s).

Other features, objects, and advantages of the invention will be apparent from the description, drawings and from the claims. The following examples are incorporated to demonstrate preferred embodiments of the invention.

### EXAMPLES

### MATERIALS & METHODS

### Viruses and cells

All recombinant viruses used in this study are double deleted thymidine kinase (J2R) and ribonucleotide reductase (I4L) vaccinia viruses derived from the Copenhagen strain. VVTK-RR-/GFP is the double deleted vaccinia virus expressing the gene marker *GFP* (for Green Fluorescent Protein). VVTK-RR-/CDD1 is the double deleted vaccinia virus expressing the yeast cytidine deaminase CDD1 gene (Kurtz et al., 1999, Curr. Genet., 36(3):130-6). VVTK-RR-/hCD is the double deleted vaccinia virus expressing the human cytidine deaminase *CDA* cDNA (Laliberté et Momparler, 1994, Cancer Res., 54(20):5401-7). VVTK-RR-/APOBEC2 is the double deleted vaccinia virus expressing the human APOBEC2 gene. *GFP, CCD1, hCD* and *APOBEC2* genes are inserted in the thymidine kinase locus and placed under the control of the p11K.5 promoter. Virus structures were confirmed by multiple PCRs. Final recombinant vaccinia viruses were amplified in primary chicken embryo fibroblasts (CEF) and virus stocks were titrated on CEF by plaque assay.

Human colon cancer cell lines LoVo (ATCC CCL-229), MIA PaCa-2 (ATCC CRL-1420) and HCT 116 (ATCC CCL-247) were obtained from the American Type Culture Collection (ATCC, Rockville, MD). Human esophagus cancer cell line OE-19 was obtained from European Collection of Cell Culture (ECACC 96071721). All human tumor cell lines were grown in Dulbecco's modified Eagle's medium (DMEM) supplemented with 10% fetal calf serum (FCS). Primary chicken embryo fibroblasts (CEF) were used for recombination, amplification and titration of viral vectors. CEF cells were prepared from chicken embryos obtained from fertilized eggs (Charles River SPAFAS) previously incubated 11 or 12 days at 37°C in a humid atmosphere. Chicken embryos were dissected and treated with a 2.5% (w/v) solution of trypsin. CEF cells were maintained in Eagle-based Medium (MBE) supplemented with 5 % fetal calf serum.

### Western blot analysis.

Human tumor pancreatic carcinoma MIA PaCa-2 cells and human colorectal carcinoma LoVo cells were infected by VVTK-RR-and VV-TK-RR-/hCD at a MOI of 0.1 and incubated for 24 hours. Thirty µg of cell lysate proteins were run on a 10% SDS/PAGE gel under reducing conditions and transferred onto a nitrocellulose membrane. The membrane was incubated with rabbit polyclonal antibody human hCD (Abcam), washed, and incubated with secondary antibody coupled horseradish peroxidase (Amersham). Signal detection was done by enhanced chemiluminescence (Amersham).

### In vivo antitumor activity

Female Swiss nude mice were obtained from Charles River Laboratories. Animals used in the studies were uniform in age (6 weeks) and body weight (20-23 g).

To evaluate the therapeutic activity of vaccinia vectors in human xenograft tumor model, 5 × 10⁶ human cancer cells (LoVo, HCT-116 or OE-19) were injected subcutaneously (s.c.) into the flank of the mice. When tumors reached a diameter of 70-100 mm³, the mice were randomized in a blinded manner and treated with the indicated vectors.

Nude mice bearing established s.c. LoVo tumors were infected once intravenously (tail vein injection) with the indicated vectors at dose of 1×10⁷ PFU.

Nude mice bearing established s.c. HCT-116 tumors were infected once intravenously (tail vein injection) with the indicated vectors at dose of 1×10⁵ PFU.

Nude mice bearing established s.c. OE-19 tumors were infected once intravenously (tail vein injection) with the indicated vectors at dose of 1×10⁶ PFU or 5×10⁵ PFU.

Tumor size was measured twice a week using calipers. Tumor volumes were calculated in mm³ using the formula (*Π*/6) (length × width²).

### Liquid chromatography-high resolution mass spectrometry analysis

This study was performed by the "Hospices Civils de Lyon" for the quantification of intracellular nucleotides and nucleosides in human HCT-116 and LoVo tumor cells. The analytical methodology and methodology for the preparation of the samples are described in Machon et al., 2015, J. Chromatogr. A. ; 1405:116-25 and in Machon et al., 2014, Anal. Bioanal. Chem. ; 406(12):2925-41. The used mass spectrometer was a Q Exactive™ Plus (ThermoFicher), a high-resolution and orbitrap type detector.

1.5 × 10⁶ HCT-116 and LoVo cells plated in T-25 flasks were infected by VVTK-RR-/GFP and VVTK-RR-/hCD at an MOI of 0.01, and incubated at 37°C in the presence of 5% C0₂. 36 hours later for HCT-116 and 60 hours later for LoVo, culture medium was removed and cells were washed three times with cold PBS before adding 3 ml of cold methanol/water (70/30, v/v). After 5 min of incubation, the supernatant was stored at - 80°C until analysis.

Nucleotides and nucleosides concentrations were evaluated 36 hours post-infection in HCT-116, and 60 hours post-infection in LoVo. Cytidine and uridine were quantified.

### RESULTS

### Virus engineering

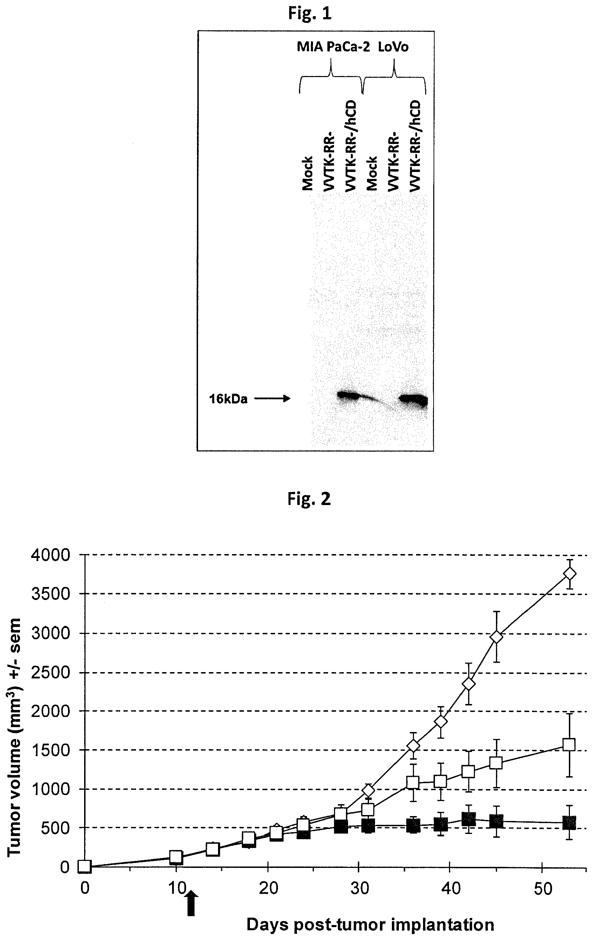

The expression of the human CDA (hCD) protein in the vaccinia virus was confirmed by Western blot using the rabbit polyclonal antibody human CDA (Figure 1). Western blot shows that VVTK-RR-/hCD expressed the expected 16 kDa CDA on MiaPaca-2 and LoVo cells.

### Antitumor activity in multiple tumor models of VVTK-RR- expressing cytidine deaminase

The ability of VVTK-RR- expressing cytidine deaminase to function as an oncolytic virus was examined in different models of human cancers.

We have first compared the oncolytic activity of the double deleted virus expressing *GFP* and the double deleted virus expressing *CDD1* in the colorectal LoVo model, a model which is weakly permissive to vaccinia virus oncolysis. Mice bearing LoVo tumors were injected i.v. with both viruses at 1 × 10⁷ PFU. As shown in Figure 2, a single i.v. injection of VVTK-RR-/GFP resulted in an inhibition of tumor growth compared with the control group (vehicle alone). Moreover, the anti-tumoral activity of the virus expressing the cytidine deaminase was significantly superior to the anti-tumoral activity of the virus expressing the marker gene *GFP* indicating that the yeast cytidine deaminase gene improves the therapeutic efficacy of the oncolytic vaccinia virus.

To further characterize the antitumoral efficacy of VVTK-RR-/CDD1, we tested the oncolytic activity of this virus, in comparison to VVTK-RR-/GFP, in human tumor model xenografts which are more sensitive to W oncolysis. Both viruses were injected once i.v. at 1 × 10⁵ PFU and 1 × 10⁶ PFU in HCT-116 and OE-19 models, respectively. As shown in Figure 3 and Figure 4, in both models, a single i.v. injection of VVTK-RR-/GFP induced a weak antitumor effect compared to control and this effect was dramatically increased by a single i.v. injection of VVTK-RR-/CDD1.

The antitumor effect of the double deleted vaccinia virus expressing the human cytidine deaminase gene (VVTK-RR-/hCD) was also evaluated in the OE-19 model. We performed a single i.v. injection of VVTK-RR-/hCD and VVTK-RR-/GFP at 5 × 10⁵ PFU. As shown in Figure 5, a single i.v. injection of the virus expressing the human cytidine deaminase gene resulted also in strong inhibition of tumor growth compared to virus expressing the gene marker *GFP.*

These data indicated that cytidine deaminases from different origin (yeast and human) increase the antitumor activity of oncolytic vaccinia vectors.

### Antitumor activity in LoVo tumor model of VVTK-RR- expressing FCU1

The anti-tumor activity provided by the encoded CDAse enzyme was compared to that provided by another nucleoside pool modulator in the same context. Here, the product used for the comparison is a TK and RR double deleted vaccinia virus (VVTK-RR-FCU1) encoding a FCU1 gene. The so-called FCU1 gene is obtained from the fusion of the yeast cytosine deaminase and uracil phosphoribosyltransferase sequences. The cytosine deaminase, which is a nucleoside pool modulator, catalyzes the transformation of the 5-FC prodrug into the toxic 5-FU. VVTK-RR-FCU1 was evaluated in the LoVo tumor model as well as VVTK-RR- control. As shown in Figure 6, a single i.v. injection of VVTK-RR- or VVTK-RR-FCU1 virus without 5-FC (prodrug) administration resulted in a similar and weak inhibition of tumor growth compared with the vehicle alone. This indicates that without prodrug treatment, the FCU1-encoded cytosine deaminase and uracil phosphoribosyltransferase nucleoside pool modulators do not cause an anti-tumoral effect at least in the LoVo tumor model. In contrast, the cytidine deaminase-expressing vaccinia virus does not require any prodrug administration to be effective in anti-tumor protection.

### In vivo antitumor activity of VVTK-RR- expressing APOBEC2

We have compared the oncolytic activity of the double deleted virus expressing *GFP* and the double deleted virus expressing *APOBEC2* in the colorectal HCT-116 model. Mice bearing HCT-116 tumors were injected i.v. with both viruses at 1 × 10⁵ PFU. As shown in Figure 7, a single i.v. injection of VVTK-RR-/GFP resulted in an inhibition of tumor growth compared with the control group (vehicle alone). Moreover, the anti-tumoral activity of the virus expressing APOBEC2 was significantly superior to the anti-tumoral activity of the virus expressing the marker gene GFP indicating that the APOBEC2 gene improves the therapeutic efficacy of the oncolytic vaccinia virus.

**Quantification of intracellular cytidine and uridine in human HCT-116 and LoVo tumor cells.**

**Table 1: Quantification of intracellular nucleosides 36 hours post-infection in HCT-116**

| | **Cytidine (C)** | **Uridine (U)** | **C/U** |
|---|---|---|---|
| **control** | 0.182 | 0.097 | 1.876 |
| **VVTK-RR-** | 0.149 | 0.093 | 1.602 |
| **VVTK-RR-/hCD** | 0.020 | 0.098 | 0.204 |

The results represent the chromatographic peaks surface ratio between the endogenous cytidine or uridine, and the corresponding internal standards. The results are expressed per 1 million of cells.

This evaluation shows that in cells infected with the control or with the empty virus (VVTK-RR-), the levels of ratio C/U, comprised between 1.60 and 1.88, are similar. In comparison, the cells infected with the virus coding for cytidine deaminase (VVTK-RR-/hCD) show an abnormal level of ratio C/U (0.204). This low ratio indicates that WTK-RR-/hCD provokes a nucleosides pool disequilibrium.

**Table 2 : Quantification of intracellular nucleosides 60 hours post-infection In LoVo**

| | **Cytidine (C)** | **Uridine (U)** | **C/U** |
|---|---|---|---|
| **control** | 0.056 | 0.021 | 2.667 |
| **VVTK-RR-** | 0.071 | 0.029 | 2.448 |
| **WTK-RR-/hCD** | - | 0.039 | - |

The results represent the chromatographic peaks surface ratio between the endogenous cytidine or uridine, and the corresponding internal standards. The results are expressed per 1 million of cells. Cytidine was not detected in LoVo cells 36h after VVTK-RR-/hCD infection.

This evaluation shows that in cells infected with the control or with the empty virus (VVTK-RR-), the levels of ratio C/U, comprised between 2.44 and 2.67, are similar. In comparison, cytidine was not detected in LoVo cells infected with the virus coding for cytidine deaminase (VVTK-RR-/hCD). Considering a limit of detection of 0.02 (the lowest level of cytidine detected in the present experiments, see Table 1) the ratio C/U would be less than 0.51. This low ratio confirms that VVTK-RR-/hCD provokes a nucleosides pool disequilibrium.

### BIBLIOGRAPHIC REFERENCES

Adra et al., 1987, Gene 60: 65-74
Boviatsis et al., Gene Ther. 1: 323-31
Broyles et al., 1993, Virol. 195: 863-5
Buckland et al., 2014 Dec, PLoS Genet., 10(12):e1004846
Buller et al., 1985, Nature, 317(6040):813-5
Caroll et al., 1997, Virology 238: 198-211
Carrey et al., 2011, PLoS ONE, 6(7) e22442)
Carrey et al., 2014, Sci Rep 4: 6154 doi 10.1038
Chambers et al., 1995, Proc. Natl. Acad. Sci. USA 92: 1411-5
Chakrabarti et al., 1997, Biotechniques, 23:1094-7
Chernajovsky et al., 2006, BMJ, 332(7534):170-2
Conticello et al., 2005, Mol Biol Evol. 22:367-77
Dayhoffed, 1981, Suppl., 3: 482-9
De Pas et al., 2008, J. Clin. Oncol., 20;26:14644
Demontis et al., 1998, Biochim. Biophys. Acta. 1443:323-33
Erbs et al., 2000, Cancer Res., 60(14):3813-22
Erbs et al., 2008, Cancer Gene Ther. 15(1): 18-28
Fallaux et al., 1998, Human Gene Ther. 9: 1909-17
Fisher et al., 2006, Curr. Opin. Mol. Ther., 8(4):301-13
Fitzgerald et al., 2006, Hum. Genet., 119(3):276-83
Foloppe et al., 2008, Gene Ther., 15:1361-71
Freeman et al., 2006, Mol. Ther. 13(1):221-8)
Geevarghese et al., 2010, Hum. Gene Ther. 21(9): 1119-28
Gemble et al., 2015, PLoS Genet, 11(7) e1005384
Graham et al., 1997, J. Gen. Virol. 36: 59-72
Guse et al., 2011, Expert Opinion Biol. Ther.11(5): 595-608
Hammond et al., 1997, J. Virol. Methods, 66: 135-8
Harrington et al., 2015, Expert Rev. Anticancer Ther. 15(12):1389-1403
Hermiston et al., 2006, Curr. Opin. Mol. Ther., 8(4):322-30
Hidefumi et al., 2014, Biomed. Rep., 2(3):392-395
Kaufmann et al., 2013, J. Invest. Dermatol. 133(4): 1034-42
Khuri et al., 2000, Nat. Med 6(8): 879-85
Kirn et al., 2001, Nat. Med. 7: 781
Kohnken et al., 2015, Mol. Cancer; 14:176
Kremer et al., 2006, Virol. J., 3:86
Kumar and Boyle, 1990, Virology, 179: 151-8
Kurtz et al., 1999, Curr. Genet., 36(3):130-6
Laliberté et Momparler, 1994, Cancer Res., 54(20):5401-7
Lorence et al., 2007, Curr. Cancer Drug Targets 7(2):157-67
Machon et al., 2014, Anal. Bioanal. Chem. ; 406(12):2925-41
Machon et al., 2015, J. Chromatogr. A. ; 1405:116-25
Mameri et al., 2016, Clin. Cancer. Res. DOI: 10.1158/1078-0432.CCR-16-0626
Martuza et al., 1991, Science 252: 854-6
McDonald et al., 2006; Breast Cancer Treat. 99(2): 177-84
Mineta et al., 1994, Cancer Res. 54: 3363-66
Moris et al., 2014, Front Microbiol, 5:534
Needleman et Wunsh, J.Mol. Biol. 48,443-453, 1970
Oliva et al., 2016, Immunol. Cell. Biol. 94:689-700
Pastor-Anglada et al, 2015, Front. Pharmacol., 6(13):1-14
Phuangsab et al., 2001, Cancer Lett. 172(1):27-36
Pyles et al., 1994, J. Virol. 68: 4963-72
Rudin et al., 2011, Clin. Cancer. Res. 17(4): 888-95
Somasekaram et al., 1999, J. Biol. Chem. 274 (40):28405-12
Stojdl et al., 2000, Nat. Med. 6(7): 821-5
Stojdl et al., 2003, Cancer Cell 4(4): 263-75
Thorne et al., 2005, Curr. Opin. Mol. Ther., 7(4):359-65
Tibaldi et al, 2012, Ann. Oncol., 23:670-677
Van der Maaden et al., 2012, J. Control release 161: 645-55
Vieira et al., 2013, Biomed. Res. Int., 2013:ID 683095
Wong et al., 2010, Viruses 2: 78-106
Xia et al., 2004, Ai Zheng 23(12): 1666-70
Zauri et al., 2015, Nature, 524:114-118
Zhang et al., 2007, Cancer Res. 67:10038-46
US6,054,438
US 8,394,385
US 8,772,023
WO98/02522
WO98/04727
WO98/10088
WO98/37095
WO98/56415
WO01/66137
WO03/053463
WO2005/042728
WO2006/108846
WO2007/056847
WO2007/147528
WO2007/147529
WO2008/114021
WO2008/129058
WO2008/138533
WO2009/065546
WO2009/065547
WO2009/100521
WO2010118013
WO2010118010
WO2010/130753
WO2010/130756
WO2011/128704
WO2012/001075
WO2013/022764
WO2014/063832

## Claims

1. An oncolytic poxvirus defective in the J2R locus comprising a nucleotide sequence encoding a cytidine deaminase (CDAse) polypeptide, preferably selected from the group consisting of CDA, CDD, EC 3.5.4.5, APOBEC, AID and cytidine deaminase-like proteins.

2. The oncolytic poxvirus of claim 1, wherein said APOBEC is selected from the group consisting of APOBEC1, APOBEC2, APOBEC3A, APOBEC3B, APOBEC3C, APOBEC3D, APOBEC3E, APOBEC3F, APOBEC3G, APOBEC3H and APOBEC4, and preferably wherein said APOBEC is APOBEC2.

3. The oncolytic poxvirus of claim 1, wherein said CDAse is a yeast cytidine deaminase (CDD1), preferably a CDD1 comprising an amino acid sequence having at least 80%, preferably greater than 90%, and more preferably greater than 95% identity with SEQ ID NO:1, or a human cytidine deaminase (hCD), preferably a hCD comprising an amino acid sequence having at least 80%, preferably greater than 90%, and more preferably greater than 95% identity with SEQ ID NO:2.

4. The oncolytic poxvirus of claim 2, wherein said APOBEC2 comprises an amino acid sequence having at least 80%, preferably greater than 90%, and more preferably greater than 95% identity with SEQ ID NO:3.

5. The oncolytic poxvirus of any of claims 1 to 4, wherein said CDAse polypeptide catalyses the deamination of cytidine-based components, preferably cytidine-based components that are free or complexed, to uridine-based components, like cytidine to uridine and deoxycytidine (dC) to deoxyuridine (dU).

6. The oncolytic poxvirus of claim 1, wherein said poxvirus is a poxvirus belonging to the Orthopoxvirus genus, wherein said poxvirus preferably belongs to the vaccinia virus (VV) species, preferably selected from the group consisting of Copenhagen, Wyeth and Western Reserve (WR), or to the cowpox virus species.

7. The oncolytic poxvirus of claim 1, wherein said virus is further defective in the I4L and/or F4L locus.

8. The oncolytic poxvirus of claim 7, wherein said poxvirus is a vaccinia virus defective in viral TK and RR encoded activities which encodes a human or yeast CDAse.

9. The oncolytic poxvirus of any of claims 6 to 8, wherein said virus further comprises one or more nucleic acid(s) of interest, said nucleic acid of interest preferably encoding an immunostimulatory polypeptide, an antigen or a permease.

10. The oncolytic poxvirus of any of claims 1 to 9, wherein said virus further comprises the elements necessary for the expression of said cytidine deaminase and said one or more nucleic acid(s) of interest.

11. A process for preparing an oncolytic poxvirus, in which process:
(i) An oncolytic poxvirus according to anyone of claims 1 to 10 is introduced into a producer cell;
(ii) said producer cell is cultured under conditions which are appropriate for enabling said oncolytic poxvirus to be produced, and;
(iii) said oncolytic poxvirusis recovered from the cell culture.

12. A composition comprising the oncolytic poxvirus of any one of claims 1 to 10, or prepared according to claim 11, which further comprises a pharmaceutically acceptable vehicle, wherein said composition preferably comprises a dose of oncolytic virus comprised between 10⁶ and 5×10⁹ PFU, and wherein said oncolytic virus is preferably formulated for parenteral route administration with a preference for intravenous or intratumoral route.

13. The oncolytic poxvirus of any one of claims 1 to 10, or prepared according to claim 11, or the composition of claim 12, for use in the treatment of a disease or a pathologic condition in a subject in need thereof.

14. The oncolytic poxvirus of any one of claims 1 to 10, or prepared according to claim 11, or the composition of claim 12, for use in the prophylaxis or the treatment of a proliferative disease, said proliferative disease preferably being selected from the group consisting of cancer, tumor and restenosis, wherein said cancer is preferably a gastro-intestinal cancer, preferably comprising a cancer of oesophagus, gallbladder, liver, pancreas, stomach, small intestine, bowel (large intestine or colon and rectum), or anus.

15. The oncolytic poxvirus for use according to claim 14, further comprising the administration of one or more substances effective in anticancer therapy.

## Patentansprüche

1. Onkolytisches Pockenvirus, das im J2R-Locus defektiv ist, umfassend eine Nucleotidsequenz, die ein Cytidindeamniase (CDAse)-Polypeptid codiert, bevorzugt ausgewählt aus der Gruppe bestehend aus CDA, CDD, EC 3.5.4.5, APOBEC, AID und Cytidin-ähnlichen Proteine (cytidine deaminase-like proteins).

2. Onkolytisches Pockenvirus nach Anspruch 1, wobei das APOBEC ausgewählt ist aus der Gruppe bestehend aus APOBEC1, APOBEC2, APOBEC3A, APOBEC3B, APOBEC3C, APOBEC3D, APOBEC3E, APOBEC3F, APOBEC3G, APOBEC3H und APOBEC4 und bevorzugt wobei das APOBEC APOBEC2 ist.

3. Onkolytisches Pockenvirus nach Anspruch 1, wobei die CDAse eine Hefe-Cytidindeaminase (CDD1), bevorzugt eine CDD1 ist, die eine Aminosäuresequenz umfasst, die mindestens 80%, bevorzugt mehr als 90% und stärker bevorzugt mehr als 95% Identität mit SEQ ID NO:1 aufweist, oder eine humane Cytidindeaminase (hCD), bevorzugt eine hCD ist, die eine Aminosäuresequenz umfasst, die mindestens 80%, bevorzugt mehr als 90% und stärker bevorzugt mehr als 95% Identität mit SEQ ID NO:2 aufweist.

4. Onkolytisches Pockenvirus nach Anspruch 2, wobei das APOBEC2 eine Aminosäuresequenz umfasst, die mindestens 80%, bevorzugt mehr als 90% und stärker bevorzugt mehr als 95% Identität mit SEQ ID NO:3 aufweist.

5. Onkolytisches Pockenvirus nach einem der Ansprüche 1 bis 4, wobei das CDAse-Polypeptid die Deaminierung von Komponenten mit Cytidinbasis, bevorzugt Komponenten mit Cytidinbasis, die frei oder komplexiert sind, zu Komponenten mit Uridinbasis katalysiert, wie z.B. Cytidin zu Uridin und Deoxycytidin (dC) zu Deoxyuridin (dU).

6. Onkolytisches Pockenvirus nach Anspruch 1, wobei das Pockenvirus ein zur Gattung Orthopockenvirus gehörendes Pockenvirus ist, wobei das Pockenvirus bevorzugt zur Art Vacciniavirus (VV), bevorzugt ausgewählt aus der Gruppe bestehend aus Copenhagen, Wyeth und Western Reserve (WR), oder zur Art der Kuhpockenviren gehört.

7. Onkolytisches Pockenvirus nach Anspruch 1, wobei das Virus des Weiteren im I4L- und/oder F4L-Locus defektiv ist.

8. Onkolytisches Pockenvirus nach Anspruch 7, wobei das Pockenvirus ein in viralen TK- und RR-codierten Aktivitäten defektives Vacciniavirus ist, das eine humane oder eine Hefe-CDAse codiert.

9. Onkolytisches Pockenvirus nach einem der Ansprüche 6 bis 8, wobei das Virus des Weiteren eine oder mehrere Nucleinsäure(n) von Interesse umfasst, wobei die Nucleinsäure von Interesse bevorzugt ein immunstimulatorisches Polypeptid, ein Antigen oder eine Permease codiert.

10. Onkolytisches Pockenvirus nach einem der Ansprüche 1 bis 9, wobei das Virus des Weiteren die für die Expression der Cytidindeaminase erforderlichen Elemente und das eine oder die mehreren Nucleinsäure(n) von Interesse umfasst.

11. Verfahren zur Herstellung eines onkolytischen Pockenvirus, wobei in diesem Verfahren:
(i) ein onkolytisches Pockenvirus nach einem der Ansprüche 1 bis 10 in eine Erzeugerzelle (producer cell) eingebracht wird;
(ii) die Erzeugerzelle unter Bedingungen kultiviert wird, die geeignet sind, die Herstellung des onkolytischen Pockenvirus zu ermöglichen; und
(iii) das onkolytische Pockenvirus aus der Zellkultur gewonnen wird.

12. Zusammensetzung, umfassend das onkolytische Pockenvirus nach einem der Ansprüche 1 bis 10 oder hergestellt nach Anspruch 11, die des Weiteren ein pharmazeutisch verträgliches Vehikel umfasst, wobei die Zusammensetzung bevorzugt eine Dosis an onkolytischem Virus umfasst, die zwischen 10⁶ und 5×10⁹ PFU liegt, und wobei das onkolytische Virus bevorzugt für die Verabreichung über die parenterale Route, mit Präferenz für die intravenöse oder intratumorale Route formuliert ist.

13. Onkolytisches Pockenvirus nach einem der Ansprüche 1 bis 10 oder hergestellt nach Anspruch 11, oder Zusammensetzung nach Anspruch 12, zur Verwendung bei der Behandlung einer Erkrankung oder eines pathologischen Zustands bei einem Individuum, das diese benötigt.

14. Onkolytisches Pockenvirus nach einem der Ansprüche 1 bis 10 oder hergestellt nach Anspruch 11, oder Zusammensetzung nach Anspruch 12, zur Verwendung bei der Vorbeugung oder der Behandlung einer proliferativen Erkrankung, wobei die proliferative Erkrankung bevorzugt ausgewählt ist aus der Gruppe bestehend aus Krebs, Tumor und Restenose, wobei die Krebserkrankung bevorzugt Magen-Darm-Krebs ist, bevorzugt umfassend Krebserkrankungen von Speiseröhre, Gallenblase, Leber, Bauchspeicheldrüse, Magen, Dünndarm, Darm (Dickdarm oder Colon und Rektum) oder Anus.

15. Onkolytisches Pockenvirus zur Verwendung nach Anspruch 14, die des Weiteren die Verabreichung einer oder mehrerer in der Anti-Krebstherapie wirksamen Substanzen umfasst.

## Revendications

1. Poxvirus oncolytique défectueux en regard du locus J2R comprenant une séquence de nucléotides codant un polypeptide de cytidine désaminase (CDAse), de préférence choisi dans le groupe constitué par CDA, CDD, EC 3.5.4.5, APOBEC, AID et des protéines analogues à la cytidine désaminase.

2. Poxvirus oncolytique selon la revendication 1, dans lequel ladite APOBEC est choisie dans le groupe constitué par les APOBEC1, APOBEC2, APOBEC3A, APOBEC3B, APOBEC3C, APOBEC3D, APOBEC3E, APOBEC3F, APOBEC3G, APOBEC3H et APOBEC4, et de préférence dans lequel ladite APOBEC est APOBEC2.

3. Poxvirus oncolytique selon la revendication 1, dans lequel ladite CDAse est une cytidine désaminase de levure (CDD1), de préférence une CDD1 comprenant une séquence d'acides aminés ayant une identité d'au moins 80 %, de préférence supérieure à 90 %, et mieux encore supérieure à 95 % avec la SEQ ID NO : 1, ou une cytidine désaminase humaine (hCD), de préférence une hCD comprenant une séquence d'acides aminés ayant une identité d'au moins 80 %, de préférence supérieure à 90 %, et mieux encore supérieure à 95 % avec la SEQ ID NO : 2.

4. Poxvirus oncolytique selon la revendication 2, dans lequel ladite APOBEC2 comprend une séquence d'acides aminés ayant une identité d'au moins 80 %, de préférence supérieure à 90 %, et mieux encore supérieure à 95 % avec la SEQ ID NO : 3.

5. Poxvirus oncolytique selon l'une quelconque des revendications 1 à 4, dans lequel ledit polypeptide de CDAse catalyse la désamination de composants à base de cytidine, de préférence de composants à base de cytidine qui sont libres ou complexés, en des composants à base d'uridine, telle que de la cytidine en uridine et de la désoxycytidine (dC) en désoxyuridine (dU).

6. Poxvirus oncolytique selon la revendication 1, lequel poxvirus est un poxvirus appartenant au genre Orthopoxvirus, lequel poxvirus appartient de préférence à l'espèce du virus de la vaccine (VV), de préférence choisie dans le groupe constitué par Copenhague, Wyeth et Western Reserve (WR), ou à l'espèce du virus de la variole des vaches.

7. Poxvirus oncolytique selon la revendication 1, lequel virus est en outre défectueux en regard des locus I4L et/ou F4L.

8. Poxvirus oncolytique selon la revendication 7, lequel poxvirus est un virus de vaccine défectueux en regard des activités virales codées par TK et RR, qui code une CDAse humaine ou de levure.

9. Poxvirus oncolytique selon l'une quelconque des revendications 6 à 8, lequel virus comprend en outre un ou plusieurs acides nucléiques d'intérêt, ledit acide nucléique d'intérêt codant de préférence un polypeptide immunostimulant, un antigène ou une perméase.

10. Poxvirus oncolytique selon l'une quelconque des revendications 1 à 9, lequel virus comprend en outre les éléments nécessaires à l'expression de ladite cytidine désaminase et lesdits un ou plusieurs acides nucléiques d'intérêt.

11. Procédé pour préparer un poxvirus oncolytique, procédé dans lequel :
(i) un poxvirus oncolytique selon l'une quelconque des revendications 1 à 10 est introduit dans une cellule productrice ;
(ii) ladite cellule productrice est cultivée dans des conditions qui sont appropriées pour permettre la production dudit poxvirus oncolytique ; et
(iii) ledit poxvirus oncolytique est récupéré à partir de la culture cellulaire.

12. Composition comprenant le poxvirus oncolytique de l'une quelconque des revendications 1 à 10, ou préparé selon la revendication 11, qui comprend en outre un véhicule pharmaceutiquement acceptable, laquelle composition comprend de préférence une dose de virus oncolytique comprise entre 10⁶ et 5 × 10⁹ PFU, et dans laquelle ledit virus oncolytique est de préférence formulé pour une administration par voie parentérale avec une préférence pour la voie intraveineuse ou intratumorale.

13. Poxvirus oncolytique selon l'une quelconque des revendications 1 à 10, ou préparé selon la revendication 11, ou la composition selon la revendication 12, pour une utilisation dans le traitement d'une maladie ou d'un état pathologique chez un sujet en ayant besoin.

14. Poxvirus oncolytique selon l'une quelconque des revendications 1 à 10, ou préparé selon la revendication 11, ou la composition selon la revendication 12, pour une utilisation dans la prophylaxie ou le traitement d'une maladie proliférative, ladite maladie proliférative étant de préférence choisie dans le groupe constitué par un cancer, une tumeur et une resténose, dans lequel ledit cancer est de préférence un cancer gastro-intestinal, de préférence comprenant un cancer de l'œsophage, de la vésicule biliaire, du foie, du pancréas, de l'estomac, de l'intestin grêle, des intestins (gros intestin ou côlon et rectum), ou de l'anus.

15. Poxvirus oncolytique pour une utilisation selon la revendication 14, comprenant en outre l'administration d'une ou plusieurs substances efficaces en thérapie anticancéreuse.
